# EUROPÄISCHE PATENTANMELDUNG

(11) **EP 1 949 887 A2**
(43) Veröffentlichungstag der Anmeldung: **30.07.2008**
(21) Anmeldenummer: 07023503.1
(22) Anmeldetag: 05.12.2007
(51) Int. Cl.: A61K 8/49, A61K 8/44, A61K 8/42, A61K 8/64, A61K 8/73, A61K 8/67, A61K 8/34, A61K 8/97, A61Q 19/06

(54) **Kosmetische Zusammensetzungen zur Glättung und Straffung der Haut**

(30) Priorität: 27.12.2006 DE 102006062438
(71) Anmelder: Henkel AG & Co. KGaA, 40589 Düsseldorf (DE)
(72) Erfinder: Heinen, Soraya, 50858 Köln (DE); Waldmann-Laue, Marianne, 40789 Monheim (DE)

(57) **Zusammenfassung**

Die Erfindung betrifft topisch zu applizierende kosmetische Zusammensetzungen zur Straffung und Glättung der Haut, insbesondere der von Cellulite betroffenen Haut, die in einem geeigneten kosmetischen oder dermatologischen Träger eine Kombination von mindestens einem Aporphin-Alkaloid und mindestens einem weiteren Wirkstoff, ausgewählt aus Purin und Purinderivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, hyperämisierenden Wirkstoffen sowie Mischungen dieser Substanzen, enthalten.

## Beschreibung

Die vorliegende Erfindung betrifft topisch zu applizierende kosmetische Zusammensetzungen zur Straffung und Glättung der Haut, insbesondere der von Cellulite betroffenen Haut.

### Stand der Technik

Das Körperbewusstsein der Verbraucher ist in den vergangenen Jahren deutlich gestiegen, was der Anstieg des Verbrauchs von kosmetischen Zusammensetzungen zeigt. Dabei werden neben Zusammensetzungen zur reinigenden und pflegenden Anwendung zunehmend auch kosmetische Zusammensetzungen nachgefragt, die helfen, die Körpersilhouette zu verbessern. Der Behandlung der Cellulite - ein vor allem bei Frauen weit verbreitetes Phänomen - kommt dabei eine wichtige Rolle zu.
Unter Cellulite, fälschlicherweise oft Cellulitis genannt, versteht man eine nicht-entzündliche konstitutionell (geschlechtstypisch) bedingte Adipositas mit leichter Lymphstauung und geringer (mucoider) Ödembildung im Bereich des Bindegewebes (sogenannte Adipositas circumscripta oedematosa). Cellulite kommt besonders bei Frauen in der Hüft-, Oberschenkel- und Glutealregion vor. Meist sind ein so genanntes "Matratzenphänomen" (durch Bindegewebssepten netzartig eingezogene Oberfläche) sowie das so genannte "Orangenhaut-Phänomen" (trichterförmige Follikeleinziehungen nach Kneifen) erkennbar. Das sichtbare Bild der Cellulite beruht auf einer Zunahme von Fettpolstern in der Subcutis (Unterhautfettgewebe), einer Bindegewebsschwäche sowie einer Minderung der Durchströmungsverhältnisse in den Blut- und Lymphbahnen. Für das Auftreten der Cellulite wird eine Funktionsminderung des Gefäßsystems als wesentliche Ursache verantwortlich gemacht. Weitere Ursachen sind eine Turgorminderung (Herabsetzung des osmotischen Drucks in den Zellen) sowie eine zum Teil anlagebedingte Schwäche des Bindegewebes mit gleichzeitigem Auftreten von vergrößerten Fettzellkammern, häufig, aber nicht ausschließlich, infolge von Übergewicht, unausgewogener Ernährung und Bewegungsmangel. Die Subcutis stellt ein lockeres Bindegewebe dar mit Einlagerungen von Fettzellengruppen (Fettläppchen). Ihrem Aufbau gemäß ist die Subcutis Verschiebe- und Verbindungsschicht zwischen Haut und Unterlage, Nährstoff- und Wasserspeicher, Sitz der Druckrezeptoren, Ort des Fett- und Kohlenhydrat-Stoffwechsels und Durchgangsgebiet für größere Gefäße zur Hautoberfläche. Die Entstehung der Cellulite kann ferner auf eine erhöhte Durchlässigkeit der Haargefäßwände zurückgeführt werden, die das Eindringen von Wasser ins Bindegewebe erlaubt. Daneben kann an den betroffenen Hautbereichen ein lokaler Mangel an Testosteron bestehen.

Mittlerweile sind eine Vielzahl von Zusammensetzungen und Behandlungsmethoden gegen Cellulite bekannt. Grundlage für die vorgeschlagenen Behandlungsansätze bildet die Erkenntnis, dass die Mikrozirkulationsstörungen im Stadium der Cellulite in der Hautschicht alle Stoffwechselprozesse und Syntheseleistungen nachfolgender Zellpopulationen, besonders die der Fibroblasten im Corium und der epidermalen Zellen, beeinträchtigen. Ziel der Behandlungen muss es danach sein, eine ausreichende Vaskularisierung und Versorgung der Subcutis wiederherzustellen. Um dies zu erreichen, wurden diverse Massagesysteme entwickelt, bei denen beispielsweise eine physikalische Behandlung der Hautoberfläche mit Hilfe eines kleinen Massagegerätes, gegebenenfalls unter gleichzeitigem Einsatz von ausgesuchten Phytoextrakten, erfolgt.
Darüber hinaus sind Kombinationen einer mechanischen und wirkstofflichen Einwirkung auf die von Cellulite betroffenen Hautpartien bekannt. Dabei werden die jeweiligen Körperteile, insbesondere Oberschenkel, Hüfte und Gesäß, unter Anwendung spezieller Übungsgeräte einer dynamischen isometrischen Einwirkung unterworfen, während diese Körperteile einen Verband tragen, der mit einer Lösung eines mineralischen Stoffes, zum Beispiel Meerschlamm, getränkt ist. Mit dieser Behandlung sollen bestimmte Mineralien aus Fettablagerungen des Körpers extrahiert werden, um das Elastin des Weichgewebes zu revitalisieren. Ein Nachteil solcher mechanischer Behandlungsmethoden liegt darin, dass ein durch kräftige Massagen erzeugter, externer Druck die Zellen irritiert und vielfältige Reaktionen der Hautzellen hervorruft. Als Folge produzieren die Zellen verstärkt Elastase und Collagenase. Diese das Stützgewebe abbauenden Enzyme lassen das Bindegewebe eher erschlaffen, als es zu straffen.
Neben Massagesystemen sind diverse Kosmetika im Markt erhältlich, die Anti-Cellulite-Wirkstoffe enthalten, wie beispielsweise Seetang-Extrakte oder fettabbauende Enzyme. Weitere im Stand der Technik gegen Cellulite erhältliche Zusammensetzungen enthalten als Wirkstoffe Extrakte von Elizabethae, einer Korallenart, von Heidekraut, die Entzündungen im Gewebe und damit die Entstehung gewebe-schwächender Enzyme bekämpfen sollen, sowie Algenbestandteile, die die Fettverbrennung aktivieren sollen; daneben sollen beispielsweise Centella Asiatica, Milchproteine, Coenzym Q 10 oder Vitamin A die geschwächte Collagen- und Elastinproduktion stärken. Diese Wirkstoffe werden häufig mit Fruchtsäuren kombiniert, um die Haut zu glätten.
Mittel und Behandlungsmethoden zur Straffung und Glättung der Haut sind eine bedeutende kosmetische Herausforderung. Bei diesen Mitteln besteht ein Bedarf nach Inhaltsstoffen, die den Zubereitungen weiteren Nutzen verleihen, sei es aus anwendungstechnischer Sicht, aus herstelltechnischer Sicht oder aus Sicht des Verbrauchers, der beispielsweise aufgrund bestimmter Inhaltstoffe ein verbessertes subjektives Empfinden dem kosmetischen Mittel gegenüber entwickelt. Der Schwerpunkt der Leistung eines Inhaltsstoffes kann dabei je nach kosmetischem Mittel auch unterschiedlich sein. So kann ein und derselbe Stoff in einem Shampoo oder einer Spülung pflegende Eigenschaften für die behandelten Haare bewirken, während er in einem Haarfärbemittel darüber hinaus die Anfärbung der Kopfhaut verringert oder die zum Teil aggressive Wirkung des Färbemittels kompensiert.

### Aufgabenstellung

Eine Aufgabe der vorliegenden Erfindung war es, die Nachteile des Standes der Technik zu beseitigen. Insbesondere war es eine Aufgabe der vorliegenden Erfindung, kosmetische Zusammensetzungen zur Verfügung zu stellen, die eine Verbesserung des Erscheinungsbildes der von Cellulite betroffenen Hautpartien bewirken können.
Eine weitere Aufgabe der vorliegenden Erfindung war es, verbesserte kosmetische Zusammensetzungen zur Verfügung zu stellen, die die Mikrozirkulation in der Haut anregen.
Eine weitere Aufgabe der vorliegenden Erfindung war es, verbesserte kosmetische Zusammensetzungen zur Verfügung zu stellen, die die Festigkeit der Haut verbessern.
Eine weitere Aufgabe der vorliegenden Erfindung war es, verbesserte kosmetische Zusammensetzungen zur Verfügung zu stellen, die die Lipolyse in der Subcutis unterstützen und/oder die Lipogenese in der Subcutis inhibieren.
Eine weitere Aufgabe der vorliegenden Erfindung war es, verbesserte kosmetische Zusammensetzungen zur Verfügung zu stellen, die die Mikrozirkulation in der Haut anregen und gleichzeitig die Festigkeit der Haut verbessern.
Eine weitere Aufgabe der vorliegenden Erfindung war es, verbesserte kosmetische Zusammensetzungen zur Verfügung zu stellen, die die Mikrozirkulation in der Haut anregen und gleichzeitig die Lipolyse in der Subcutis unterstützen und/oder die Lipogenese in der Subcutis inhibieren.
Eine weitere Aufgabe der vorliegenden Erfindung war es, verbesserte kosmetische Zusammensetzungen zur Verfügung zu stellen, die die Festigkeit der Haut verbessern und gleichzeitig die Lipolyse in der Subcutis unterstützen und/oder die Lipogenese in der Subcutis inhibieren.
Eine weitere Aufgabe der vorliegenden Erfindung war es, verbesserte kosmetische Zusammensetzungen zur Verfügung zu stellen, die die Mikrozirkulation in der Haut anregen, die Festigkeit der Haut verbessern und gleichzeitig die Lipolyse in der Subcutis unterstützen und/oder die Lipogenese in der Subcutis inhibieren.

Überraschend wurde gefunden, dass topische kosmetische oder dermatologische Zusammensetzungen, die in einem geeigneten kosmetischen oder dermatologischen Träger eine Kombination von mindestens einem Aporphin-Alkaloid der allgemeinen Formel (APO-ALK-I) und mindestens einem weiteren Wirkstoff, ausgewählt aus Purin und Purinderivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, hyperämisierenden Wirkstoffen sowie Mischungen dieser Substanzen, enthalten, die gestellte Aufgabe in hervorragender Weise lösen.

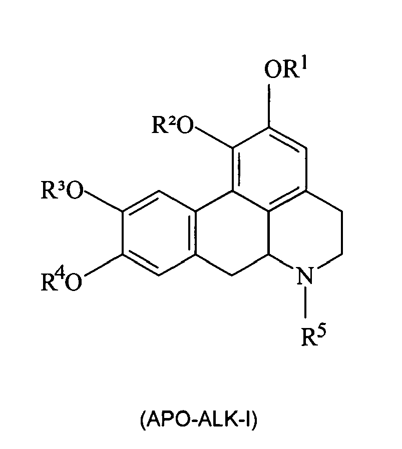

Gegenstand der vorliegenden Erfindung sind kosmetische oder dermatologische Zusammensetzungen zur topischen Behandlung der Haut, die in einem geeigneten kosmetischen oder dermatologischen Träger mindestens ein Aporphin-Alkaloid und mindestens einen weiteren Wirkstoff, ausgewählt aus
a) Purin und Purin-Derivaten,
b) natürlichen Betainverbindungen,
c) Harnstoff und alkyl- oder hydroxyalkylsubstituierten Harnstoffen,
d) Monomeren, Oligomeren und Polymeren von Aminosäuren, N-C₂-C₂₄-Acylaminosäuren und/oder den Estern und/oder den physiologisch verträglichen Salzen dieser Substanzen,
e) Polysacchariden,
f) hyperämisierenden Wirkstoffen,
g) sowie Mischungen dieser Wirkstoffe,
enthalten.

Erfindungsgemäß bevorzugte Aporphin-Alkaloide sind ausgewählt ist aus Verbindungen der allgemeinen Struktur (APO-ALK-I), 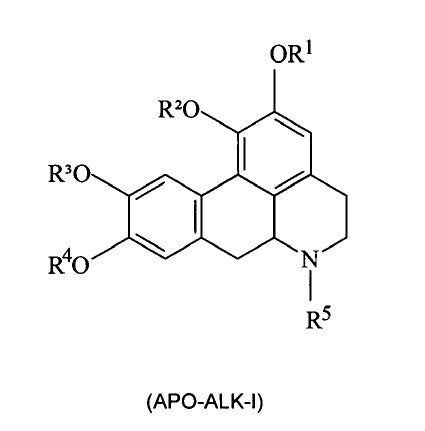 in der die Reste R¹, R², R³, R⁴ und R⁵, die gleich oder verschieden sein können, ausgewählt sind aus einem Wasserstoffatom, einem Halogenatom, einem C₁- bis C₄-Alkylrest, einem C₁- bis C₄-Monohydroxyalkylrest, einem C₂- bis C₄-Polyhydroxyalkylrest, einem (C₁- bis C₄)-Alkoxy-(C₁- bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁-bis C₄)-aminoalkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe, einer Aryl-C₁-C₆-alkylgruppe, einer Acylgruppe, einer Sulfonylgruppe oder einem Zucker, und den kosmetisch verträglichen Salzen von (APO-ALK-I) mit einer Säure, in Form aller 15 optischen Isomere, in isomerenreiner Form oder als Mischung optischer Isomere.
Beispiele für die als Substituenten in den erfindungsgemäß kombinierten Verbindungen genannten C₁-C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁-C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für bevorzugte Halogensubstituenten sind erfindungsgemäß F-, Cl- oder Br-Atome, Cl-Atome sind ganz besonders bevorzugt.
Als Zuckerrest können beliebige Mono- oder Oligo-, insbesondere Disaccharide, eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Besonders bevorzugte Aporphin-Alkaloide sind ausgewählt aus Verbindungen der allgemeinen Struktur (APO-ALK-I), in denen R¹ = R² = R³ = R⁴ = R⁵ = CH₃ ist. Diese Verbindung wird auch als 1,2,9,10-Tetramethoxyaporphin bezeichnet.

Bevorzugte erfindungsgemäße Zusammensetzungen enthalten mindestens ein Aporphin-Alkaloid in engeren Mengenbereichen. Hier sind erfindungsgemäß bevorzugte kosmetische Zusammensetzungen dadurch gekennzeichnet, dass sie - bezogen auf ihr Gewicht - eine Gesamtmenge von 0,0001 - 1 Gew.-%, vorzugsweise 0,001 - 0,5 Gew.-%, besonders bevorzugt 0,002 - 0,1 Gew.-% und insbesondere 0,005 - 0,01 Gew.-% mindestens eines Aporphin-Alkaloids enthalten.

Als einen bevorzugten weiteren wesentlichen Inhaltsstoff enthalten die erfindungsgemäßen Zusammensetzungen Purin und/oder mindestens ein Derivat des Purins. Purin (7*H*-Imidazo[4,5-*d*]pyrimidin) kommt frei in der Natur nicht vor, bildet jedoch den Grundkörper der Purine. Purine ihrerseits sind eine Gruppe wichtiger, in der Natur weit verbreiteter und an menschlichen, tierischen, pflanzlichen und mikrobiellen Stoffwechselvorgängen beteiligter Verbindungen, die sich vom Grundkörper durch Substitution mit OH, NH₂, SH in 2-, 6- und 8-Stellung und/oder mit CH₃ in 1-, 3-, 7-Stellung ableiten. Purin kann beispielsweise aus Aminoacetonitril und Formamid hergestellt werden. Purine und Purinderivate werden oft aus Naturstoffen isoliert, sind aber auch auf vielen Wegen synthetisch zugänglich.

Bevorzugte erfindungsgemäße Zusammensetzungen enthalten Purin und/oder Purinderivat(e) in engeren Mengenbereichen. Hier sind erfindungsgemäß bevorzugte kosmetische Zusammensetzungen dadurch gekennzeichnet, dass sie - bezogen auf ihr Gewicht - eine Gesamtmenge von 0,001 - 2,5 Gew.-%, bevorzugt 0,01 - 1 Gew.-%, besonders bevorzugt 0,1 - 0,8 Gew.-% und insbesondere 0,2 - 0,5 Gew.-% Purin(e) und/oder Purinderivat(e) enthalten.

Unter Purin, den Purinen und den Purinderivaten sind einige Vertreter erfindungsgemäß besonders bevorzugt. Erfindungsgemäß bevorzugte kosmetische Zusammensetzungen sind dadurch gekennzeichnet, dass sie Purin und/oder Purinderivat(e) der Formel (PUR-1) enthalten, 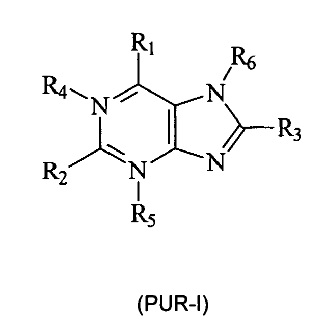 in der die Reste R¹, R² und R³ unabhängig voneinander ausgewählt sind aus -H, - OH, -NH₂ und - SH und die Reste R⁴, R⁵ und R⁶ unabhängig voneinander ausgewählt sind aus -H, einem C₁- bis C₄-Alkylrest, einem C₁- bis C₄-Monohydroxyalkylrest, einem C₂- bis C₄-Polyhydroxyalkylrest, einem (C₁- bis C₄)-Alkoxy-(C₁- bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁-bis C₄)-aminoalkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe und einer Aryl-C₁-C₆-alkylgruppe.

Erfindungsgemäß besonders bevorzugte kosmetische Zusammensetzungen sind dadurch gekennzeichnet, dass sie Purin und/oder Purinderivat(e) der Formel (PUR-I) enthalten, in der die Reste R¹, R² und R³ unabhängig voneinander ausgewählt sind aus -H, - OH, -NH₂, -SH und die Reste R⁴, R⁵ und R⁶ unabhängig voneinander ausgewählt sind aus -H, -CH₃ und -CH₂-CH₃, wobei folgende Verbindungen außerordentlich bevorzugt sind:
- Purin (R¹ = R² = R³ = R⁴ = R⁵ = R⁶ = H)
- Adenin (R¹ = NH₂, R² = R³ = R⁴ = R⁵ = R⁶ = H)
- Guanin (R¹ = OH, R² = NH₂, R³ = R⁴ = R⁵ = R⁶ = H)
- Harnsäure (R¹ = R² = R³ = OH, R⁴ = R⁵ = R⁶ = H)
- Hypoxanthin (R¹ = OH, R² = R³ = R⁴ = R⁵ = R⁶ = H)
- 6-Purinthiol (R¹ = SH, R² = R³ = R⁴ = R⁵ = R⁶ = H)
- 6-Thioguanin (R¹ = SH, R² = NH₂, R³ = R⁴ = R⁵ = R⁶ = H)
- Xanthin (R¹ R² = OH, R³ = R⁴ = R⁵ = R⁶ = H)
- Coffein (R¹ = R² = OH, R³ = H, R⁴ = R⁵ = R⁶ = CH₃)
- Theobromin (R¹ = R² = OH, R³ = R⁴ = H, R⁵ = R⁶ = CH₃)
- Theophyllin (R¹ = R² = OH, R³ = H, R⁴ = CH₃, R⁵ = CH₃, R⁶ = H).

Verbindungen der allgemeinen Formel (PUR-I), in denen R¹ und/oder R² einer OH-Gruppe entsprechen, existieren in tautomeren Verbindungen, insbesondere solchen der allgemeinen Formel (PUR-II), ausgehend von (PUR-I) mit R¹ = R² = OH : 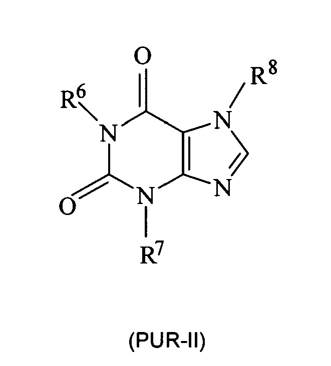 wobei in der Formel (PUR-II) die Reste R⁶, R⁷ und R⁸, die gleich oder verschieden sein können, bevorzugt ausgewählt sind aus einem Wasserstoffatom, einem C₁- bis C₄-Alkylrest, einem C₁- bis C₄-Monohydroxyalkylrest, einem C₂- bis C₄-Polyhydroxyalkylrest, einem (C₁- bis C₄)-Alkoxy-(C₁- bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁-bis C₄)-aminoalkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe und einer Aryl-C₁-C₆-alkylgruppe, wobei Coffein (R⁶ = R⁷ = R⁸ = CH₃), Theobromin (R⁶ = H, R⁷ = R⁸ = CH₃) und Theophyllin (R⁶ = R⁷ = CH₃, R⁸ = H) außerordentlich bevorzugt sind.

Je nach gewünschtem Anwendungszweck der kosmetischen Zusammensetzung kann dabei die Art und Menge des Purinderivates variieren. In haarkosmetischen Formulierungen und in Körperpflegemitteln zur Anticellulite-Behandlung der Haut, hat sich insbesondere Coffein bewährt, das beispielsweise in Shampoos vorzugsweise in Mengen von 0,005 bis 0,25 Gew.-%, weiter bevorzugt von 0,01 bis 0,1 Gew.-% und insbesondere von 0,01 bis 0,05 Gew.-% (jeweils bezogen auf das Shampoo) und in Anticellulite-Mitteln, insbesondere Cremes, Gelen und Lotionen, vorzugsweise in Mengen von 0,01 - 2 Gew.-%, weiter bevorzugt 0,1 - 1,5 Gew.-% und besonders bevorzugt 0,2 - 0,8 Gew.-%, jeweils bezogen auf das Mittel, eingesetzt werden kann.

Weitere bevorzugte erfindungsgemäße Zusammensetzungen sind dadurch gekennzeichnet, dass sie zusätzlich zu dem mindestens einen Aporphin-Alkaloid als weiteren wesentlichen Inhaltsstoff mindestens eine natürliche Betainverbindung enthalten. Erfindungsgemäß eingesetzte natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl.
Bevorzugte erfindungsgemäße Zusammensetzungen sind dadurch gekennzeichnet, dass sie mindestens eine natürliche Betainverbindung in einer Gesamtmenge von 0,01 bis 5 Gew.-%, bevorzugt 0,1 bis 3 Gew.-%, besonders bevorzugt 0,2 bis 1 Gew.-% und außerordentlich bevorzugt 0,3 - 0,5 Gew.-%, jeweils bezogen auf die gesamte Zusammensetzung, enthalten.

Weitere bevorzugte erfindungsgemäße Zusammensetzungen sind dadurch gekennzeichnet, dass sie zusätzlich zu dem mindestens einen Aporphin-Alkaloid als weiteren wesentlichen Inhaltsstoff mindestens eine Substanz enthalten, die ausgewählt ist aus Harnstoff und alkyl- oder hydroxyalkylsubstituierten Harnstoffen.
Erfindungsgemäß bevorzugte alkyl- oder hydroxyalkylsubstituierte Harnstoffverbindungen weisen die allgemeine Formel (UREA-I) auf, 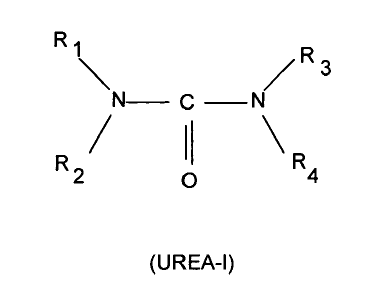 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.
Erfindungsgemäß besonders bevorzugte alkyl- oder hydroxyalkylsubstituierte Harnstoffverbindungen der allgemeinen Formel (UREA-I) sind ausgewählt aus Verbindungen, bei denen jeweils mindestens einer der Reste R₁ und R₂ bzw. R₃ und R₄ eine C₂-C₆-Hydroxyalkyl-Gruppe, die mit 1 bis 5 Hydroxylgruppen oder C₁-C₄-Hydroxyalkyl-Gruppen substituiert ist, darstellt. Besonders bevorzugte C₂-C₆-Hydroxyalkyl-Gruppen, die mit 1 bis 5 Hydroxylgruppen oder C₁-C₄-Hydroxyalkyl-Gruppen substituiert sind, sind ausgewählt aus 2-Hydroxyethyl-, 3-Hydroxypropyl-, 2-Hydroxypropyl, 2-Hydroxyisopropyl- und 4-Hydroxybutyl-Gruppen, wobei die 2-Hydroxyethyl-Gruppe außerordentlich bevorzugt ist. Ebenfalls außerordentlich bevorzugt ist Bis-N,N'-(2-Hydroxyethyl)-harnstoff. Harnstoff selbst ist ebenfalls besonders bevorzugt. Weiterhin bevorzugt ist es, Mischungen von Harnstoff und alkyl- oder hydroxyalkylsubstituierten Harnstoffverbindungen einzusetzen. Außerordentlich bevorzugt sind Mischungen von Harnstoff und Bis-N,N'-(2-Hydroxyethyl)-harnstoff.

Weitere bevorzugte erfindungsgemäße Zusammensetzungen sind dadurch gekennzeichnet, dass sie zusätzlich zu dem mindestens einen Aporphin-Alkaloid als weiteren wesentlichen Inhaltsstoff mindestens eine Substanz enthalten, die ausgewählt ist aus Monomeren, Oligomeren und Polymeren von Aminosäuren, N-C₂-C₂₄-Acylaminosäuren und/oder den Estern und/oder den physiologisch verträglichen Salzen dieser Substanzen, sowie Mischungen dieser Wirkstoffe.
Die Monomere der Aminosäuren und/oder der N-C₂-C₂₄-Acylaminosäuren sind bevorzugt ausgewählt aus Alanin, Arginin, Asparagin, Asparaginsäure, Canavanin, Citrullin, Cystein, Cystin, Dipalmitoylhydroxyprolin, Desmosin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, Zink- und Natriumpyroglutamat und Natriumlauroylglutamat.
Der C₂ - C₂₄-Acylrest, mit dem die genannten Aminosäuren an der Aminogruppe derivatisiert sind, ist bevorzugt ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten.
Mit den vorgenannten C₂ - C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift DPHP von der Firma Seppic erhältlich ist.
Die physiologisch verträglichen Salze der erfindungsgemäß bevorzugten Aminosäuren oder Aminosäurederivate sind bevorzugt ausgewählt aus den Ammonium-, Alkalimetall-, Magnesium-, Calcium-, Aluminium-, Zink- und Mangan-Salzen. Besonders bevorzugt sind die Natrium-, Kalium-, Magnesium-, Zink- und Mangan-Salze; außerordentlich bevorzugt sind die Natrium-, Kalium- und Magnesiumsalze.

Unter Aminosäureoligomeren werden erfindungsgemäß Peptide mit 2 - 30, bevorzugt 2 - 15, Aminosäuren, verstanden. Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die N-acyliert und/ oder verestert sein können. Zahlreiche dieser Aminosäureoligomere stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z.B. die Collagensynthese, induzieren, beziehungsweise sind in der Lage, an die Sequenz Arg-Phe-Lys in Thrombospondin I (TSP-1) zu binden und damit aktives TGF-β (*tissue growth factor*)*,* der die Synthese von Collagen in dermalen Fibroblasten induziert, freizusetzen. Derartige Aminosäureoligomere werden im Stand der Technik als Wirkstoffe gegen die Hautalterung verwendet. Ihr Einsatz in Kombination mit einem Aporphin-Alkaloid als Anticellulite-Mittel ist bislang nicht bekannt.
Erfindungsgemäß bevorzugte, gegebenenfalls N-acylierte und/oder veresterte Dipeptide sind Acetyl-Citrullyl-Arginin (z. B. Exsy-Algine von Exsymol mit der INCI-Bezeichnung Acetyl Citrull Amido Arginine), Tyr-Arg (Dipeptide-1), Val-Trp (Dipeptide-2), Asn-Phe, Asp-Phe, N-Palmitoyl-β-Ala-His, N-Acetyl-Tyr-Arg-hexyldecylester (z. B. Calmosensine von Sederma), Carnosin (β-Ala-His) und N-Palmitoyl-Pro-Arg. Erfindungsgemäß bevorzugte, gegebenenfalls N-acylierte und/oder veresterte Tripeptide sind Gly-His-Lys, das z. B. unter der Bezeichnung "Omega-CH-Aktivator" von der Firma GfN oder in acylierter Form (N-Palmitoyl-Gly-His-Lys) unter der Bezeichnung Biopeptide CL von Sederma erhältlich ist, aber (in acylierter Form) auch einen Bestandteil des Produktes Matrixyl 3000 von Sederma darstellt. Das Tripeptid Gly-His-Lys kann auch als Kupfersalz (Cu²⁺) eingesetzt werden und ist als solches über ProCyte Corporation zu beziehen. Weiterhin können Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und Ile geeignet. Die erfindungsgemäß bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn, oder Citrullin ersetzt. Ein weiteres erfindungsgemäß bevorzugtes Tripeptid ist Gly-His-Arg (INCl-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist; weitere erfindungsgemäß bevorzugte Tripeptide sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure), Dap-Val-Lys, Palmitoyl-Lys-Val-Lys, z. B. erhältlich von der Firma Pentapharm unter der Bezeichnung SYN^{®}-COLL, Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn, Gly-Asp-Ser, N-Elaidoyl-Lys-Phe-Lys und N-Acetyl-Arg-Lys-Arg-NH₂.
Erfindungsgemäß bevorzugte, gegebenenfalls N-acylierte und/oder veresterte Tetrapeptide sind ausgewählt aus Rigin und Rigin-basierten Tetrapeptiden sowie ALAMCAT-Tetrapeptiden. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg, das z. B. unter der Bezeichnung Eyeliss von Sederma erhältlich ist, aber auch einen Bestandteil des Produktes Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im folgenden definiert ist, ausgewählt werden. Besonders bevorzugte Rigin-basierte Tetrapetide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gln und den Aminosäuren, die Gln substituieren können, Xcc ausgewählt ist aus Pro oder Arg und den Aminosäuren, die Pro und Arg substituieren können.
Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (IIe).
Die bevorzugten Aminosäuren, die Gln ersetzen können, beinhalten eine Seitenkette mit einer Aminogruppe, die bei neutralem pH (pH 6-7) überwiegend ungeladen vorliegt, z.B. Asn, Lys, Orn, 5-Hydroxyprolin, Citrullin und Canavanin.
Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin.
Als Rigin-Analoga sind erfindungsgemäß Gly-Gln-Arg-Pro und Val-Val-Arg-Pro bevorzugt.
ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (IIe). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt.
Erfindungsgemäß bevorzugte, gegebenenfalls N-acylierte und/oder veresterte Pentapeptide sind ausgewählt aus Lys-Thr-Thr-Lys-Ser und seinen N-acylierten Derivaten, besonders bevorzugt N-Palmitoyl-Lys-Thr-Thr-Lys-Ser, das unter der Bezeichnung Matrixyl von der Firma Sederma erhältlich ist, weiterhin N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Val-Gly-Val-Ala-Pro-Gly und seine N-acylierten Derivate, besonders bevorzugt N-Palmitoyl-Val-Gly-Val-Ala-Pro-Gly, das unter der Bezeichnung Biopeptide EL von der Firma Sederma erhältlich ist, weiterhin Glu-Glu-Met-Gln-Arg-Arg, weiterhin Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec), Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Ala-Asp-Leu-Lys-Pro-Thr (Hexapeptide-3, z. B. Peptide 02 von Vincience), Val-Val-Arg-Pro-Pro-Pro, Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience), Hexapeptide-10 (z. B. Collaxyl von Vincience oder Seriseline von Lipotec) und Hexapeptide-11 (z. B. Peptamide-6 von Arch Personal Care). Ein erfindungsgemäß bevorzugtes Pentadecapeptid ist z. B. der Rohstoff Vinci 01 von Vincience (Pentadecapeptide-1). Ein weiteres bevorzugtes Aminosäureoligomer ist das Peptidderivat L-Glutamylaminoethyl-indol (enthalten in dem Rohstoff Glistin von Exsymol).
Ein erfindungsgemäß besonders bevorzugter Aminosäureoligomer-Wirkstoff ist die Kombination aus N-Palmitoyl-Gly-His-Lys und N-Palmitoyl-Gly-Gln-Pro-Arg, wie sie beispielsweise in dem Rohstoff Matrixyl 3000 von der Firma Sederma erhältlich ist.

Ein weiterer erfindungsgemäß bevorzugter Wirkstoff, deren Gegenwart eine unerwartete Wirkungssteigerung des mindestens einen Aporphin-Alkaloids der Formel (APO-ALK-I) bewirkt, ist ausgewählt aus Polymeren der Aminosäuren und/oder der N-C₂-C₂₄-Acylaminosäuren.
Erfindungsgemäß kombinierte Polymere der Aminosäuren und/oder der N-C₂-C₂₄-Acylaminosäuren sind bevorzugt ausgewählt aus pflanzlichen und tierischen Proteinhydrolysaten und/oder Proteinen. Tierische Proteinhydrolysate sind z. B. Elastin-, Collagen-, Keratin-, Seiden-, Conchiolin- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda). Besonders bevorzugt sind Sojaproteinhydrolysate, besonders bevorzugt Sojaproteinhydrolysate mit einer mittleren Molmasse (Mw) im Bereich von 1200 - 1800 Dalton, bevorzugt im Bereich von 1400 - 1700 Dalton, z. B. unter dem Handelsnamen Ridulisse C^{®} von der Firma Silab erhältlich, und Sojaproteinhydrolysate mit einer mittleren Molmasse (Mw) im Bereich von 600 - 1000 Dalton, bevorzugt 800 Dalton, z. B. unter dem Handelsnamen Phytokine^{®} von Coletica erhältlich. mit Kokosfettsäuren N-acylierten und/oder veresterten Sojaproteinhydrolysaten in Form ihrer Alkalimetallsalze. Kokosfettsäuren umfassen überwiegend Alkancarbonsäuren mit einer Anzahl an Kohlenstoffatomen von 8 - 18, insbesondere Caprylsäure, Caprinsäure, Laurinsäure, Myristinsäure, Palmitinsäure und Stearinsäure. Bevorzugte Alkalimetallsalze sind ausgewählt aus Lithium-, Natrium- und Kaliumsalzen, wobei die Kaliumsalze besonders bevorzugt sind.
Ein erfindungsgemäß besonders bevorzugtes Sojaproteinhydrolysat mit einer mittleren Molmasse (Mw) im Bereich von 1200 - 1800 Dalton, bevorzugt im Bereich von 1400 - 1700 Dalton (Da), ist unter dem Handelsnamen Ridulisse C von der Firma Silab erhältlich. Der Proteinhydrolysat-Anteil in Ridulisse C enthält vier Molmassenfraktionen: 0,8 Gew.-% mit einer Mw > 12.500 Da, 22,2 Gew.-% mit 1355 Da < Mw < 12.500 Da, 43,1 Gew.-% 75 Da < Mw < 1355 Da und 33,9 Gew.-% < 75 Da.

Ein weiteres, erfindungsgemäß besonders bevorzugtes Sojaproteinhydrolysat ist ein mit Kokosfettsäuren N-acyliertes und/oder verestertes Sojaproteinhydrolysat in Form des Kaliumsalzes, das unter der Handelsbezeichnung Coccopolipeptide di Soja von der Firma Sinerga erhältlich ist.
Weiterhin erfindungsgemäß bevorzugt sind Keratinhydrolysate, insbesondere Wollkeratinhydrolysate. Ein besonders bevorzugtes Wollkeratinhydrolysat ist unter der Bezeichnung Keratec Pep von der Firma Croda erhältlich. Keratec Pep weist eine kleinere Molmassenfraktion mit einer durchschnittlichen Molmasse von 150 Dalton und eine größere Molmassenfraktion mit einer durchschnittlichen Molmasse von 1265 Dalton auf. Weiterhin erfindungsgemäß bevorzugt sind Conchiolinhydrolysate, insbesondere solche, die unter den Bezeichnungen Pearl Protein Extract und Pearl Protein Extract BG von der Firma Maruzen erhältlich sind. Conchiolin ist ein komplexes Protein, das aus dem äußeren Epithelium von Mollusken, insbesondere von Perlmuscheln und diversen Schneckenarten, erzeugt wird und das durch Einlagerung von Calciumcarbonat-Kristallen die sehr stabile Schale dieser Mollusken bildet.
Proteinhydrolysate können naturgemäß auch monomere Aminosäuren und Oligopeptide enthalten; ihre Zusammensetzung ist normalerweise nicht definiert.
Ein erfindungsgemäß besonders bevorzugtes Weizenproteinhydrolysat ist unter dem Handelsnamen Liftiline von der Firma Silab erhältlich.
Ebenfalls bevorzugt ist der Einsatz von Acylderivaten der Proteinhydrolysate, z. B. in Form ihrer Fettsäure-Kondensationsprodukte. Entsprechende Handelsprodukte sind z. B. Lamepon^{®} (Cognis), Gluadin^{®} (Cognis), Lexein^{®} (Inolex), Crolastin^{®} oder Crotein^{®} (Croda).

Erfindungsgemäß bevorzugt sind auch kationisierte Proteinhydrolysate. Besondes bevorzugt sind kationische Proteinhydrolysate, deren zugrunde liegender Proteinanteil eine Molmasse von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Weiterhin können die kationischen Proteinhydrolysate auch noch weiter derivatisiert sein. Als typische Beispiele für erfindungsgemäß bevorzugte kationische Proteinhydrolysate und -derivate sind einige der unter den INCI- Bezeichnungen im "International Cosmetic Ingredient Dictionary and Handbook", (seventh edition 1997, The Cosmetic, Toiletry, and Fragrance Association 1101 17^{th} Street, N.W., Suite 300, Washington, DC 20036-4702) genannten und im Handel erhältlichen Produkte aufgeführt: Cocodimonium Hydroxypropyl Hydrolyzed Collagen, Steardimonium Hydroxypropyl Hydrolyzed Collagen, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl. Ganz besonders bevorzugt sind die kationischen Proteinhydrolysate und -derivate auf pflanzlicher Basis.

In einer weiteren bevorzugten Ausführungsform sind die erfindungsgemäß kombinierten Polymeren der Aminosäuren ausgewählt aus DNA-Reparaturenzymen.
Erfindungsgemäß bevorzugte DNA-Reparaturenzyme sind Photolyase und T4 Endonuclease V, letztere im Weiteren mit "T4N5" abgekürzt. Diese beiden Enzyme sind im Stand der Technik bereits als so genannte DNA-Reparatur-Enzyme bekannt. Unter DNA-Reparatur ist definitionsgemäß die Spaltung bzw. Entfernung von UV-induzierten Pyrimidindimeren aus der DNA zu verstehen.
Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans,* einem phototrophen marinen Mikroorganismus. Die Photolyase aus *A. nidulans* wird in technisch relevanten Mengen mittlerweile aus E. coli gewonnen. Photolyase ist zur Aktivierung auf Licht angewiesen.
Das Enzym T4 Endonuclease V wird vom *den*V-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.
Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosome^{™}, liposomenverkapselte T4N5 z. B. unter der Bezeichnung Ultrasome^{™} von der Firma AGI Dermatics, USA, erhältlich.
Erfindungsgemäß besonders bevorzugte Zusammensetzungen sind dadurch gekennzeichnet, dass sie neben dem mindestens einen Aporphin-Alkaloid der Formel (APO-ALK-1) mindestens eines der Handelsprodukte Photosomes^{™} oder Ultrasomes^{™} in Gesamtmengen von 0,1 - 10 Gew.-%, bevorzugt 0,5 - 5,0 Gew.-% und besonders bevorzugt 1,0 -4,0 Gew.-%, bezogen auf die gesamte erfindungsgemäße Zusammensetzung, enthalten.

Erfindungsgemäß besonders bevorzugte kosmetische oder dermatologische Zusammensetzungen sind dadurch gekennzeichnet, dass sie mindestens ein Sojaproteinhydrolysat mit einer mittleren Molmasse (Mw) im Bereich von 1200 - 1800 Dalton, bevorzugt im Bereich von 1400 - 1700 Dalton, in einer Gesamtmenge von 0,0001 - 5 Gew.-%, bevorzugt 0,001 - 1 Gew.-%, besonders bevorzugt 0,01 - 0,1 Gew.-% und außerordentlich bevorzugt 0,02 - 0,05 Gew.-%, enthalten, jeweils bezogen auf den Gehalt an Aktivsubstanz in der gesamten Zusammensetzung.

Erfindungsgemäß besonders bevorzugte kosmetische oder dermatologische Zusammensetzungen sind dadurch gekennzeichnet, dass sie mindestens ein Sojaproteinhydrolysat mit einer mittleren Molmasse (Mw) im Bereich von 600 - 1000 Dalton, bevorzugt 800 Dalton, in einer Gesamtmenge von 0,0001 - 1 Gew.-%, bevorzugt 0,001 - 0,1 Gew.-%, besonders bevorzugt 0,005 - 0,05 Gew.-% und außerordentlich bevorzugt 0,006 - 0,01 Gew.-%, enthalten, jeweils bezogen auf den Gehalt an Aktivsubstanz in der gesamten Zusammensetzung.

Erfindungsgemäß besonders bevorzugte kosmetische oder dermatologische Zusammensetzungen sind dadurch gekennzeichnet, dass sie mindestens ein Weizenproteinhydrolysat in einer Gesamtmenge von 0,001 - 5,0 Gew.-%, bevorzugt 0,01 - 1,0 Gew.-%, besonders bevorzugt 0,1 - 0,5 Gew.-% und außerordentlich bevorzugt 0,2 - 0,4 Gew.-%, enthalten, jeweils bezogen auf den Gehalt an Aktivsubstanz in der gesamten Zusammensetzung.

Erfindungsgemäß besonders bevorzugte kosmetische oder dermatologische Zusammensetzungen sind dadurch gekennzeichnet, dass sie zusätzlich zu dem mindestens einen Aporphin-Alkaloid der Formel (APO-ALK I) a) mindestens ein Sojaproteinhydrolysat mit einer mittleren Molmasse im Bereich von 1200 - 1800 Dalton, bevorzugt im Bereich von 1400 - 1700 Dalton b) mindestens ein Sojaproteinhydrolysat mit einer mittleren Molmasse im Bereich von 600 - 1000 Dalton, bevorzugt 800 Dalton, und c) mindestens ein Weizenproteinhydrolysat in Gewichtsverhältnissen von 1 : (0,1 - 1) : (1 - 50), bevorzugt 1 : (0,2 - 0,6) : (10 - 20), enthalten.

Erfindungsgemäß besonders bevorzugte kosmetische oder dermatologische Zusammensetzungen sind dadurch gekennzeichnet, dass sie neben dem mindestens einen Aporphin-Alkaloid der Formel (APO-ALK-I) mindestens einen kosmetischen Wirkstoff, der ausgewählt ist aus Monomeren, Oligomeren und Polymeren von Aminosäuren, N-C₂-C₂₄-Acylaminosäuren und/oder den Estern und/oder den physiologisch verträglichen Salzen dieser Substanzen, in einer Gesamtmenge von 0,00001 - 2 Gew.-%, bevorzugt 0,0001 - 1 Gew.-%, besonders bevorzugt 0,001 - 0,1 Gew.-% und außerordentlich bevorzugt 0,01 - 0,05 Gew.-%, jeweils bezogen auf den Gehalt an Aktivsubstanz in der gesamten Zusammensetzung, enthalten.

Ein weiterer erfindungsgemäß bevorzugter Wirkstoff, dessen Gegenwart eine unerwartete Wirkungssteigerung des mindestens einen Aporphin-Alkaloids der Formel (APO-ALK-I) bewirkt, ist ausgewählt aus Polysacchariden. Erfindungsgemäß bevorzugte Polysaccharide sind solche, die mindestens einen Desoxyzucker-Baustein, insbesondere Fucose und/oder Rhamnose, enthalten, besonders bevorzugt ausgewählt aus Substanzen mit der INCI-Bezeichnung Biosaccharide Gum-1 (z.B. in dem Handelsprodukt Fucogel^{®} von Solabia), Rhamnosoft^{®} (INCI-Bezeichnung Biosaccharide Gum-2) von Solabia, Fucogenol^{®} (INCI-Bezeichnung Biosaccharide Gum-3) von Solabia und Glycofilm^{®} (INCI-Bezeichnung Biosaccharide Gum-4) von Solabia, weiterhin Mischungen der vorgenannten, mindestens einen Desoxyzucker-Baustein enthaltenden Polysaccharide, beispielsweise der Mischung aus Biosaccharide Gum-2 und Biosaccharide Gum-3, erhältlich als Handelsprodukt Elastinol plus^{®} von Solabia. Weitere erfindungsgemäß bevorzugte Polysaccharide sind ausgewählt aus den Glycosaminoglycanen, besonders bevorzugt Hyaluronsäure, Dextran, Dextransulfat, Chondroitin-4-sulfat und Chondroitin-6-sulfat.

Weitere erfindungsgemäß bevorzugte Polysaccharide sind ausgewählt aus den Glucanen. Bei Glucanen oder Polyglucosanen handelt es sich um eine Klasse von Homopolysacchariden, deren Monomerbaustein ausschließlich Glucose ist. Das Glucose-Molekül kann α-glycosidisch oder β-glycosidisch verknüpft, unterschiedlich stark verzweigt oder linear angeordnet sein.
Beispiele für Glucane sind Cellulose, Amylose, Glycogen, Lichenin, Laminarin aus Algen, Pachyman aus Baumpilzen und Hefeglucane mit β-1,3-Bindung; Nigeran, ein aus Pilzen isoliertes Mycodextran (α-1,3-Glucan, α-1,4-Glucan) und Pustulan (β-1,6-Glucan).
Glucane sind unter anderem Bestandteil der Zellwand; sie werden von zahlreichen Mikroorganismen unter bestimmten physiologischen Bedingungen in ihr Milieu abgegeben oder auch erst dort synthetisiert. Dextran, ein primär α-1,6-gebundenes D-Glucan, ist das bedeutendste in dieser Polysaccharid-Gruppe. Weitere technisch interessante und erfindungsgemäß hervorragend geeignete Glucane sind Curdlan (β-1,3-D-Glucan), Pullulan (α-1,4-gebundenes und α-1,6-gebundenes D-Glucan) und Schizophyllan (β-1,3-Grundkette, β-1,6-Seitenkette). Glucane besitzen diverse immunmodulatorische Eigenschaften, auf Zelloberflächen des menschlichen Immunsystems befinden sich entsprechende Glucan-Rezeptoren.
Weitere erfindungsgemäß bevorzugte Polysaccharid-Wirkstoffe, deren Gegenwart eine unerwartete Wirkungssteigerung des mindestens einen Aporphin-Alkaloids der Formel (APO-ALK-I) bewirkt, sind ausgewählt aus beta-(1,3-1,4)-Glucanen. Besonders bevorzugt sind beta-(1,3-1,4)-Glucane, die zu etwa 70% beta-1,4-Glucosid-Verknüpfungen und zu etwa 30% beta-1,3-Glucosid-Verknüpfungen aufweisen. Derartige beta-(1,3-1,4)-Glucane sind bevorzugt erhältlich aus Extrakten aus Haferkörnern (Avena Sativa (Oat) Kernel Extract). Erfindungsgemäß besonders bevorzugte Extrakte aus Haferkörnern sind unter der Handelsbezeichnungen Drago Beta Glucan (02/060800) und SymGlucan von der Firma Symrise erhältlich.

Weitere erfindungsgemäß bevorzugte Polysaccharid-Wirkstoffe, deren Gegenwart eine unerwartete Wirkungssteigerung des mindestens einen Aporphin-Alkaloids der Formel (APO-ALKl) bewirkt, sind ausgewählt aus chemisch modifizierten Cellulose-Derivaten, vorzugsweise Hydroxyalkylcellulosen wie Hydroxyethylcellulose, Hydroxypropylcellulose, Hydroxypropylmethylcellulose, Methylcellulose, Hydroxymethylcellulose, Carboxymethylcellulose, weiterhin insbesondere aus Stärken sowie Stärkeabbauprodukten wie Amylose und Amylopektin, chemisch und/oder thermisch modifizierte Stärken, z. B. Hydroxypropylstärkephosphat, Dihydroxypropyldistärkephosphat oder die Handelsprodukte Dry Flo^{®}, weiterhin Chitosan und dessen Salzen und anderen Derivaten, weiterhin aus Polysacchariden, die Gums oder Gummen bilden, wie beispielsweise Guar-Gum, Xanthan-Gum, Alginate, insbesondere Natriumalginat, Gummi arabicum, Karaya-Gummi, Carrageenane, Johannisbrotkernmehl, Leinsamen-Gums, Schellack und Agar-Agar.

Weitere erfindungsgemäß bevorzugte Wirkstoffe, deren Gegenwart eine unerwartete Wirkungssteigerung des mindestens einen Aporphin-Alkaloids der Formel (APO-ALK-I) bewirkt, sind ausgewählt aus hyperämisierenden Wirkstoffen.
Erfindungsgemäß bevorzugte kosmetische oder dermatologische Zusammensetzungen sind dadurch gekennzeichnet, dass sie mindestens einen hyperämisierenden Wirkstoff enthalten, der ausgewählt ist aus
- Nicotinsäureestern, insbesondere Nicotinsäurebenzylester (Benzylnicotinat), Nicotinsäurebutoxyethylester (Nicoboxil), Nicotinsäuremethylester, Nicotinsäureethylester, Nicotinsäurehexylester, Nicotinsäureisopropylester, Nicotinsäuremyristylester, Tetrahydrofurfuryl-Nicotinat (Thurfyl-Nicotinat) und Tetrahydrofurfuryl-Nicotinat-Hydrochlorid,
- Pyridin-3-carbaldehyd (β-Pyridincarbaldehyd, Nicotinaldehyd),
- Salicylsäureestern, insbesondere Phenylsalicylat,
- Vanillylethern, insbesondere Vanillylbutylether,
- Carbonsäurevanillylamiden, insbesondere Nonivamid (Nonansäurevanillylamid),
- Scharfstoffen, insbesondere Capsaicin, Cantharidin, Piperin, Gingerol, Zingeron, Myristicin, Safrol, Allicin, Sedamin, Sacculatal, Chalciporon, Isochalciporon, Velleral, Vellerol, und Isothiocyanaten (Senfölen), insbesondere Benzylsenföl,
- Extrakten aus Scharfstoff-Pflanzen, insbesondere aus Capsicum, insbesondere aus Capsicum annuum und Capsicum frutescens, aber auch aus Capsicum chinense, Capsicum baccatum und Capsicum pubescens, weiterhin aus Mauerpfeffer und aus der Zaunrübe,
- Terpentinöl,
- sowie Mischungen hiervon.

Nachfolgend sind die erfindungsgemäß bevorzugten hyperämisierenden Wirkstoffe näher erläutert.

### Nonivamid

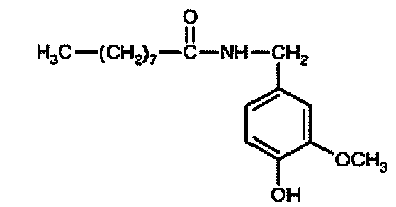

Nonivamid = Internationaler Freiname für N-(4-Hydroxy-3-methoxybenzyl)nonanamid (Pelargonsäure- oder Nonansäurevanillylamid, Pseudocapsaicin), C₁₇H₂₇NO₃, *M*ᵣ 293,40, Schmelzpunkt 52-55°C. Nonivamid ist als hyperämisierender Wirkstoff in Pflastern und Salben als Generikum im Handel.

### Vanillylbutylether

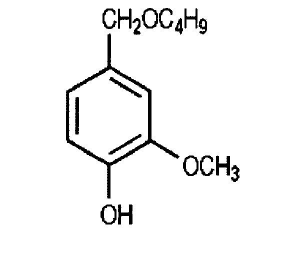

("Vanillyl..." ist erfindungsgemäß die veraltete Bezeichnung (in Anlehnung an die überholte IUPAC-Regel C-411.1) für die systematisch "(4-Hydroxy-3-methoxybenzyl)..." oder gemäß Chemical Abstracts: [(4-Hydroxy-3-methoxyphenyl)methyl]... genannte Atomgruppierung.)

Bevorzugte Scharfstoffe bzw. Scharfstoff-Pflanzen sind: Capsaicin aus Paprika, Piperin aus Pfeffer, Gingerol und Zingeron aus Ingwer, Myristicin aus Muskat, Allicin aus Knoblauch, Sedamin aus Mauerpfeffer (Sedum-Alkaloid), Sacculatal aus Lebermoosen, Chalciporon aus dem PfefferRöhrling *(Suillus piperatus),* Velleral aus Milchlings-(Lactarius)-Arten, Isothiocyanate (Senföle) aus Senf, Meerrettich, Kresse und anderen Kreuzblütlern (Brassicaceae).

### Capsaicin [(E)-N-(4-Hydroxy-3-methoxybenzyl)-8-methyl-6-nonenamid; FEMA 3404].

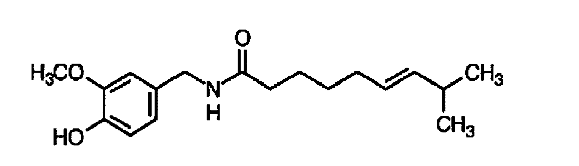

C₁₈H₂₇NO₃, *M*ᵣ 305,42. Monokline Kristalle, Schmelzpunkt 64-65°C, in Wasser kaum, in den meisten organischen Lösemitteln gut löslich. Capsaicin verursacht den scharfen Geschmack der Paprika-Früchte, Chillies und anderen Capsicum-Arten (noch in einer Verdünnung von 1:10⁵), in denen es zu 0,3 - 0,5 Gew.-% enthalten ist.

### Piperin (1-Piperoylpiperidin).

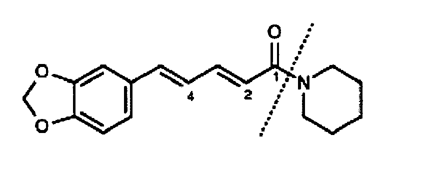

C₁₇H₁₉NO₃, *M*ᵣ 285,34, Schmelzpunkt 128-129°C. Piperin findet sich zu 5-9% im Pfeffer *(Piper nigrum*) als dessen wichtigster Scharfstoff und in vielen weiteren *Piper-*Arten (Piperaceae). Neben Piperin sind über 50 analoge Amide, meist mit Piperidin, Pyrrolidin Schmelzpunkt Isobutylamin als Basenteil aus *Piper*-Arten bekannt.

### Gingerol [6-Gingerol, 5-Hydroxy-1-(4-hydroxy-3-methoxyphenyl)-3-decanon].

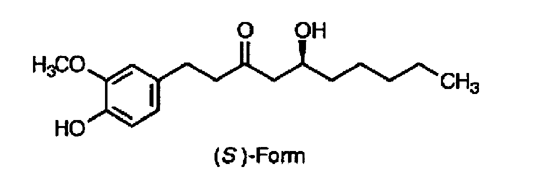

C₁₇H₂₆O₄, *M*ᵣ 294,39, (S)-Form, gelbes Öl, löslich in organischen Lösemitteln, Scharfstoff in Ingwer (*Zingiber officinale*)*.* (R)-Form, gelbes Öl. Das Racemat besitzt ähnliche Geschmackseigenschaften wie das (S)-Enantiomer, es fehlt jedoch der typische Ingwergeruch.

### Zingeron [Zingiberon, Vanillylaceton, 4-(4-Hydroxy-3-methoxyphenyl)butan-2-on; FEMA 3124]

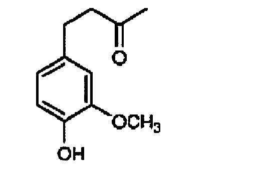

C₁₁H₁₄O₃, *M*ᵣ 194,22, farblose, scharf schmeckende Kristalle, Schmelzpunkt 40°C, wenig löslich in Wasser, löslich in Ether und verdünnten Alkalien. Zingeron ist der Scharfstoff im Oleoresin des Ingwers, aus dem es isoliert werden kann.

### Myristicin, Safrol

### [5-(2-Propenyl)-1,3-benzodioxol, 4-Allyl-1,2-methylendioxybenzol].

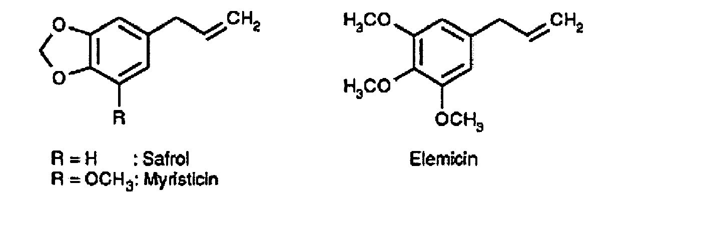

C₁₀H₁₀O₂, *M*ᵣ 162,19, farblose bis blassgelbe Flüssigkeit, Geruch nach Safran, Siedepunkt 232-234°C, Prismen, Schmelzpunkt 11°C. Safrol ist Hauptbestandteil des Sassafrasöls (bis zu 90%) und kommt in größeren Mengen auch in Campheröl vor. In zahlreichen anderen essentiellen Ölen, unter anderem in Sternanis, Basilikum, Fenchel, Anis, Zimt, schwarzem Pfeffer und in Muskatnüssen ist Safrol neben kleineren Mengen Myristicin enthalten.

*Myristicin* [4-Methoxy-6-(2-propenyl)-1,3-benzodioxol], C₁₁H₁₂O₃, *M*ᵣ 192,21, Öl, Siedepunkt 149-149,5°C (2 kPa), 95-97°C (27 Pa), löslich in Ether und Benzol, kommt unter anderem im Petersilienöl, in Möhren, in Anisöl und in der Muskatnuss vor, wo es von Safrol und *Elemicin* [1,2,3-Trimethoxy-5-(2-propenyl)benzol] begleitet ist. Myristicin verursacht den typischen Muskatnussduft.

### Allium

Die Biosynthese der Schwefel-haltigen Inhaltsstoffe führt vom Sulfat über Cystein zu Glutathion. Die SH-Gruppen dieser beiden Peptide werden mit Methacrylsäure (aus Valin) alkyliert bzw. bei Gluthathion auch methyliert.

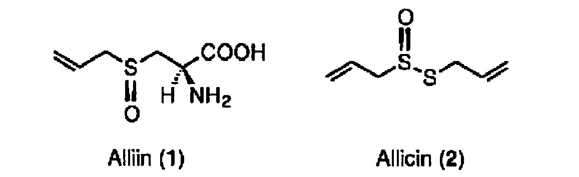

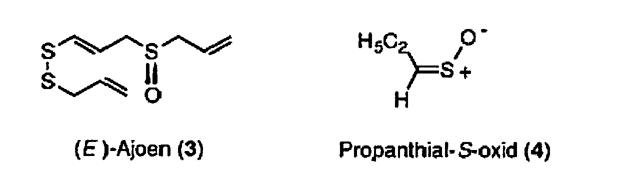

### Mauerpfeffer, Sedacin

Am C-2 monosubstituierte Piperidin-Alkaloide aus Sedum-Arten (Crassulaceae). Für den scharfen Geschmack des scharfen Mauerpfeffers *(Sedum acre)* ist *Sedamin* verantwortlich.

### Sedamin

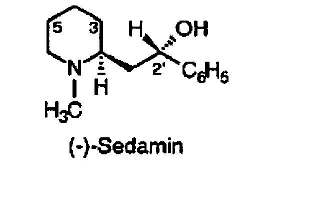

C₁₄H₂₁NO, *M*ᵣ 219,33, Schmelzpunkt 75-76°C . Mit Sedamin verwandt sind u.a. 5-Hydroxysedamin, das 2'-Epimer Allosedamin, dessen N-Demethyl-Derivat Norallosedamin und die beiden dazugehörigen Hydroxy-Derivate 3-Hydroxyallosedamin und 3-Hydroxynorallosedamin.

### Sacculatal

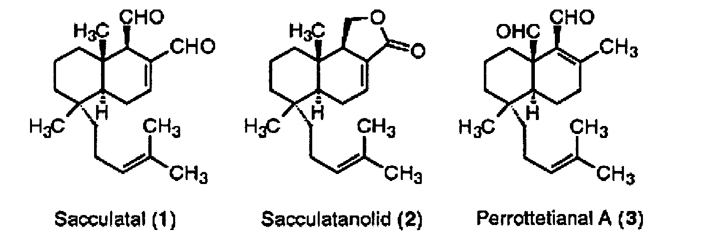

Diterpene wie *Sacculatal, Sacculatanolid* und *Perrottetianal A* (alle: C₂₀H₃₀O₂, *M*ᵣ 302,46), die das bislang nur bei Lebermoosen anzutreffende Sacculatan-Grundgerüst aufweisen. Sacculatal, Schmelzpunkt 65 -66 °C, ist für den stechend scharfen Geschmack der Thalli von *Pellia endiviifolia* und *Trichocoleopsis sacculata* mitverantwortlich.

### Chalciporon

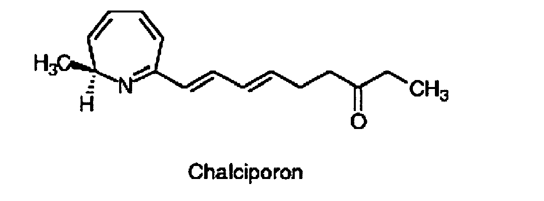

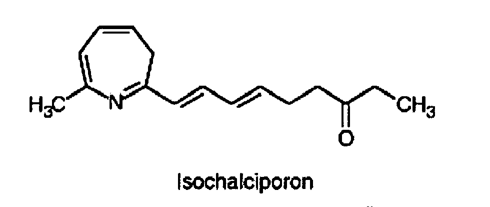

C₁₆H₂₁NO, *M*ᵣ 243,35, gelbliches Öl. Alkaloid aus dem Pfefferröhrling (*Chalciporus piperatus,* Basidiomycetes), das für den brennenden Geschmack des Pilzes verantwortlich ist. Neben Chalciporon und einigen verwandten 2H-Azepinen wurden auch die entsprechenden 3H-Azepine (z.B. *Isochalciporon,* hellgelbes Öl) isoliert, die aus den 2*H*-Isomeren durch sigmatrope [1,5]-H-Verschiebung entstehen, achiral sind und mild schmecken.

### Velleral

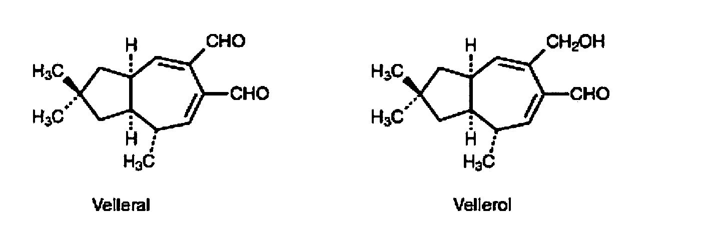

C₁₅H₂₀O₂, *M*ᵣ 232,32, Kristalle, Schmelzpunkt 86,5-87,5°C. Sesquiterpen aus Pilzen der Gattung *Lactarius* (Milchlinge), *Russula* (Täublinge) und anderen Russulaceae.

### Senföle

Historisch bedingte Bezeichnung für organische Isothiocyanate, R-N=C=S, besonders für solche, die als stechend riechende, geschmacksgebende Bestandteile der etherischen Öle vorwiegend in Brassicaceae (Kreuzblütlern) vorkommen. Die Senföle liegen dort glycosidisch gebunden als Glucosinolate vor, aus denen sie - unter Umlagerung - durch Thioglucosidasen (Beispiel: Myrosinase) freigesetzt werden.
Verbreitete Senföle sind Allylsenföl (Allylisothiocyanat), 3-Methylthiopropylsenföl, Butylsenföl, 3-Butenylsenföl, Erucin, Erysolin, Hirsutin, Isopropylsenföl, β-Phenylethylsenföl, Sulforaphan und Sulforaphen (Raphanin).

Entsprechende hyperämisierende Wirkstoffe sind im Handel erhältlich, teilweise in für kosmetische oder dermatologische Zusammensetzungen aufbereiteter Form. Erfindungsgemäß außerordentlich bevorzugte Wirkstoffe sind Ethylnicotinat, Vanillylbutylether, z.B. der Rohstoff Hotact VBE von Takasago, Capsaicin, erhältlich z. B. als Rohstoff Herbasol Extract Capsicum (INCI: Propylene Glycol, Aqua (Water), Capsicum Frutescens Fruit Extract, Sorbitol) und N-Vanillylnonamid (Pseudocapsaicin).
Bevorzugte erfindungsgemäße Zusammensetzungen sind dadurch gekennzeichnet, dass sie mindestens einen hyperämisierenden Wirkstoff in einer Aktivsubstanz-Gesamtmenge von 0,005 - 3,0 Gew.%, bevorzugt 0,01 - 1,0, besonders bevorzugt 0,05 - 0,5 Gew.% und außerordentlich bevorzugt 0,1 - 0,35 Gew.-%, jeweils bezogen auf das Gesamtgewicht der erfindungsgemäßen Zusammensetzung, enthalten.

Erfindungsgemäß besonders bevorzugte kosmetische oder dermatologische Zusammensetzungen sind dadurch gekennzeichnet, dass sie mindestens eine der folgenden Wirkstoffkombinationen enthalten:
1,2,9,10-Tetramethoxyaporphin und 1,3,7-Trimethylxanthin, denn es wurde überraschend festgestellt, dass diese Kombination die Mikrozirkulation in den Zellen der Haut verbessert und dadurch die Festigkeit der Haut verbessert,
1,2,9,10-Tetramethoxyaporphin und 1,3,7-Trimethylxanthin und Sojaproteinhydrolysat mit einer mittleren Molmasse (Mw) im Bereich von 1200 - 1800 Dalton, denn es wurde überraschend festgestellt, dass diese Kombination die Mikrozirkulation in den Zellen der Haut weiter steigern und dadurch die Festigkeit der Haut in überraschend hohem Maße verbessern kann,
1,2,9,10-Tetramethoxyaporphin und 1,3,7-Trimethylxanthin und N-Palmitoyl-Gly-His-Lys und N-Palmitoyl-Gly-Gln-Pro-Arg, denn es wurde überraschend festgestellt, dass diese Kombination die Mikrozirkulation in den Zellen der Haut weiter steigern und dadurch die Festigkeit der Haut in unerwartet hohem Maße verbessern kann,
1,2,9,10-Tetramethoxyaporphin und 1,3,7-Trimethylxanthin und N-Palmitoyl-Lys-Thr-Thr-Lys-Ser, denn es wurde überraschend festgestellt, dass diese Kombination die Mikrozirkulation in den Zellen der Haut verbessert und dadurch die Festigkeit der Haut verbessert,
1,2,9,10-Tetramethoxyaporphin und Carnitin, denn es wurde überraschend festgestellt, dass diese Kombination die Lipolyse in der Subcutis verbessert und dadurch die Festigkeit der Haut verbessert,
1,2,9,10-Tetramethoxyaporphin und 1,3,7-Trimethylxanthin und Carnitin, denn es wurde überraschend festgestellt, dass diese Kombination die Mikrozirkulation in den Zellen der Haut und die Lipolyse in der Subcutis verbessert bzw. die Lipogenese in der Subcutis hemmt und dadurch die Festigkeit der Haut in überraschend hohem Maße verbessert,
1,2,9,10-Tetramethoxyaporphin und Carnitin und Sojaproteinhydrolysat mit einer mittleren Molmasse (Mw) im Bereich von 1200 - 1800 Dalton, denn es wurde überraschend festgestellt, dass mit dieser Dreierkombination die Lipolyse in der Subcutis noch weiter gesteigert und dadurch die Festigkeit der Haut verbessert werden kann,
1,2,9,10-Tetramethoxyaporphin und Carnitin und N-Palmitoyl-Gly-His-Lys und N-Palmitoyl-Gly-Gln-Pro-Arg,
1,2,9,10-Tetramethoxyaporphin und Carnitin und N-Palmitoyl-Lys-Thr-Thr-Lys-Ser,
1,2,9,10-Tetramethoxyaporphin und 1,3,7-Trimethylxanthin und Carnitin und Sojaproteinhydrolysat mit einer mittleren Molmasse (Mw) im Bereich von 1200 - 1800 Dalton und N-Palmitoyl-Gly-His-Lys und N-Palmitoyl-Gly-Gln-Pro-Arg,
1,2,9,10-Tetramethoxyaporphin und 1,3,7-Trimethylxanthin und Carnitin und Sojaproteinhydrolysat mit einer mittleren Molmasse (Mw) im Bereich von 1200 - 1800 Dalton und N-Palmitoyl-Lys-Thr-Thr-Lys-Ser,
1,2,9,10-Tetramethoxyaporphin und 3,7-Dimethylxanthin und Sojaproteinhydrolysat mit einer mittleren Molmasse (Mw) im Bereich von 1200 - 1800 Dalton,
1,2,9,10-Tetramethoxyaporphin und 3,7-Dimethylxanthin und N-Palmitoyl-Gly-His-Lys und N-Palmitoyl-Gly-Gln-Pro-Arg,
1,2,9,10-Tetramethoxyaporphin und 3,7-Dimethylxanthin und N-Palmitoyl-Lys-Thr-Thr-Lys-Ser,
1,2,9,10-Tetramethoxyaporphin und 3,7-Dimethylxanthin und Carnitin,
1,2,9,10-Tetramethoxyaporphin und Carnitin und Sojaproteinhydrolysat mit einer mittleren Molmasse (Mw) im Bereich von 1200 - 1800 Dalton,
1,2,9,10-Tetramethoxyaporphin und Carnitin und N-Palmitoyl-Gly-His-Lys und N-Palmitoyl-Gly-Gln-Pro-Arg,
1,2,9,10-Tetramethoxyaporphin und Carnitin und N-Palmitoyl-Lys-Thr-Thr-Lys-Ser,
1,2,9,10-Tetramethoxyaporphin und 3,7-Dimethylxanthin und Carnitin und Sojaproteinhydrolysat mit einer mittleren Molmasse (Mw) im Bereich von 1200 - 1800 Dalton und N-Palmitoyl-Gly-His-Lys und N-Palmitoyl-Gly-Gln-Pro-Arg,
1,2,9,10-Tetramethoxyaporphin und 3,7-Dimethylxanthin und Carnitin und Sojaproteinhydrolysat mit einer mittleren Molmasse (Mw) im Bereich von 1200 - 1800 Dalton und N-Palmitoyl-Lys-Thr-Thr-Lys-Ser,
1,2,9,10-Tetramethoxyaporphin und 1,3-Dimethylxanthin und Sojaproteinhydrolysat mit einer mittleren Molmasse (Mw) im Bereich von 1200 - 1800 Dalton,
1,2,9,10-Tetramethoxyaporphin und 1,3-Dimethylxanthin und N-Palmitoyl-Gly-His-Lys und N-Palmitoyl-Gly-Gln-Pro-Arg,
1,2,9,10-Tetramethoxyaporphin und 1,3-Dimethylxanthin und N-Palmitoyl-Lys-Thr-Thr-Lys-Ser,
1,2,9,10-Tetramethoxyaporphin und 1,3-Dimethylxanthin und Carnitin,
1,2,9,10-Tetramethoxyaporphin und Carnitin und Sojaproteinhydrolysat mit einer mittleren Molmasse (Mw) im Bereich von 1200 - 1800 Dalton,
1,2,9,10-Tetramethoxyaporphin und Carnitin und N-Palmitoyl-Gly-His-Lys und N-Palmitoyl-Gly-Gln-Pro-Arg,
1,2,9,10-Tetramethoxyaporphin und Carnitin und N-Palmitoyl-Lys-Thr-Thr-Lys-Ser,
1,2,9,10-Tetramethoxyaporphin und 1,3-Dimethylxanthin und Carnitin und Sojaproteinhydrolysat mit einer mittleren Molmasse (Mw) im Bereich von 1200 - 1800 Dalton und N-Palmitoyl-Gly-His-Lys und N-Palmitoyl-Gly-Gln-Pro-Arg,
1,2,9,10-Tetramethoxyaporphin und 1,3-Dimethylxanthin und Carnitin und Sojaproteinhydrolysat mit einer mittleren Molmasse (Mw) im Bereich von 1200 - 1800 Dalton und N-Palmitoyl-Lys-Thr-Thr-Lys-Ser,
1,2,9,10-Tetramethoxyaporphin und Capsicum Frutescens Fruit Extrakt, denn es wurde überraschend festgestellt, dass diese Kombination die Mikrozirkulation in den Zellen der Haut und die Lipolyse in der Subcutis verbessert bzw. die Lipogenese in der Subcutis hemmt und dadurch die Festigkeit der Haut in überraschend hohem Maße verbessert,
1,2,9,10-Tetramethoxyaporphin und Ethylnicotinat, denn es wurde überraschend festgestellt, dass diese Kombination die Mikrozirkulation in den Zellen der Haut und die Lipolyse in der Subcutis verbessert bzw. die Lipogenese in der Subcutis hemmt und dadurch die Festigkeit der Haut in überraschend hohem Maße verbessert,
1,2,9,10-Tetramethoxyaporphin und Vanillylbutylether, denn es wurde überraschend festgestellt, dass diese Kombination die Mikrozirkulation in den Zellen der Haut und die Lipolyse in der Subcutis verbessert bzw. die Lipogenese in der Subcutis hemmt und dadurch die Festigkeit der Haut in überraschend hohem Maße verbessert,
1,2,9,10-Tetramethoxyaporphin und N-Vanillylnonamid, denn es wurde überraschend festgestellt, dass diese Kombination die Mikrozirkulation in den Zellen der Haut und die Lipolyse in der Subcutis verbessert bzw. die Lipogenese in der Subcutis hemmt und dadurch die Festigkeit der Haut in überraschend hohem Maße verbessert.

Es kann erfindungsgemäß besonders bevorzugt sein, dass die als Wirkstoff eingesetzten Aminosäuren und Peptide zur Verbesserung der Penetration in die Haut mit mindestens einer, bevorzugt linearen, C₂-C₂₂-Fettsäure N-acyliert und/oder verestert sind. Zur N-Acylierung und/oder Veresterung besonders bevorzugt sind C₈-C₁₈-Fettsäuren, ganz besonders bevorzugt sind Myristinsäure (C₁₄) und Palmitinsäure (C₁₆). Besonders bevorzugt ist weiterhin, dass alle verwendeten Aminosäuren und Peptide derartig N-acyliert und/oder verestert sind. Ebenfalls bevorzugt ist die Substitution der Aminosäuren und Peptide mit einer Benzyloxycarbonylgruppe an der terminalen Aminogruppe.

In einer weiteren bevorzugten Ausführungsform der Erfindung liegen die Monomeren und Oligomeren von Aminosäuren, N-C₂-C₂₄-Acylaminosäuren, den Estern und/oder den physiologisch verträglichen Metallsalzen dieser Substanzen in geträgerter Form vor, insbesondere aufgetragen auf feinteiligen, pulverförmigen Substraten wie Kieselgel, insbesondere Aerosil-Typen, weiterhin Talkum, Microsponges, modifizierten Stärken und Stärkederivaten, kristalliner Cellulose, Cellulosepulvern, Lactoglobulinderivaten, Polymerpartikeln aus Nylon, Polyolefinen, Polycarbonaten, Polyurethanen, Polyacrylaten, (Meth)acrylat- oder (Meth)acrylat-Vinyliden-Copolymeren, die vernetzt sein können, Polyestern, Polyamiden, Polystyrolen, Teflon und Siliconen. Ein besonders bevorzugter Rohstoff dieser Art sind die Vegetal Filling Spheres von Coletica.

Weitere erfindungsgemäß bevorzugte kosmetische oder dermatologische Zusammensetzungen sind dadurch gekennzeichnet, dass sie zusätzlich zu der Zweierkombination aus mindestens einem Aporphin-Alkaloid und mindestens einem weiteren Wirkstoff, ausgewählt aus Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, sowie Mischungen dieser Wirkstoffe mindestens einen weiteren, die Collagensynthese stimulierenden Wirkstoff enthalten, der ausgewählt ist aus
- Retinoiden, insbesondere Retinol und C₂-C₂₂-Fettsäureestern des Retinols,
- Kombucha,
- Olivenblattextrakten (Olea Europaea (Olive) Leaf Extract),
- Oleanolsäure,
- Oleanol,
- Grüntee-Extrakten (Camellia Sinensis),
- Kreatin,
- sowie Mischungen der vorgenannten Substanzen.
Weitere erfindungsgemäß bevorzugte Wirkstoffe, die die Collagensynthese stimulieren, sind ausgewählt aus Retinoiden. Zu den Retinoiden werden im Sinne der vorliegenden Erfindung Retinol (Vitamin A₁), die C₂-C₂₂-Fettsäureester des Retinols (= Retinylester, insbesondere Retinylpalmitat und Retinylacetat), 3,4-Didehydroretinol (Vitamin A₂), Retinal und seine Isomere, *all-trans*-Retinsäure, 9-cis-Retinsäure und 13-cis-Retinsäure (Tretinoin), die Ester der Retinsäure sowie weitere verwandte Substanzen (synthetische Retinoide) gezählt, die für ihre vielfältige biologische Wirkung insbesondere auf Wachstum und Differenzierung bekannt sind, sowie weiterhin Substanzen, die eine spezifische biologische Wirkung durch die Bindung an die Retinoid-Rezeptoren RAR (retinoid acid receptor) und RXR (retinoid X receptor) aufweisen und deren Aktivierung verursachen. Synthetische Retinoide werden in drei Gruppen eingeteilt: nichtaromatisch (z. B. *Isotretinoin),* monoaromatisch (z.B. Acitretin) und polyaromatisch (sogenannte Arotinoide, z. B. Tazarotene, die eine spezifische Wirkung auf einzelne Retinoid-Rezeptoren haben). Erfindungsgemäß besonders bevorzugte Retinoide sind Retinol und die C₂-C₂₂-Fettsäureester des Retinols, insbesondere Retinylpalmitat und Retinylacetat.

Weitere erfindungsgemäß bevorzugte Wirkstoffe, die die Collagensynthese stimulieren, sind ausgewählt aus Kombucha.
Bei Kombucha handelt es sich um ein aus dem ostasiatischen Raum stammendes, etwa 0,5 Vol.-% Alkohol enthaltendes, angenehm säuerliches, leicht süßes, moussierendes Gärungsgetränk auf Basis von Mono- oder Disaccharid-haltigem Tee (Schwarzer Tee, Grüner Tee, Kräutertee oder Früchtetee), das ohne Zusatz von Konservierungsstoffen, Farbstoffen und Aromastoffen hergestellt wird. Beim "Kombucha-Teepilz" handelt es sich tatsächlich nicht um einen Pilz, sondern um eine Symbiose von Essigsäurebakterien (meistens *Acetobacter xylinum,* aber auch *Acetobacter gluconicum)* mit verschiedenen Hefen (Saccharomyces sp., *Torula* sp., *Pichia fermentans);* fakultativ können Milchsäurebakterien vorhanden sein. Aus Gründen der Produktionssicherheit und Produktionsstabilität wird industriell gefertigter Kombucha oft wärmebehandelt.

Unter "Tee" werden erfindungsgemäß wässrige Aufgüsse von Blättern, Knospen und zarten Stielen von Thea sinensis (Camellia sinensis) und Thea assamica, aber auch von diversen anderen Pflanzen verstanden, beispielsweise Minze, Linde (Lindenblüten), Malve, Rotbusch, Kamille usw.

Bevorzugte Kombucha ist aus Mono- oder Disaccharid-haltigem Tee von fermentierten (Schwarztee, Oolong-Tee, Gelber Tee, Pu-Erh-Tee) oder unfermentierten (Grüntee) Blättern von Thea sinensis (Camellia sinensis) oder Thea assamica gewonnen. Weitere bevorzugte Kombucha ist durch Fermentation von (meist mit Saccharose) gezuckertem Tee mit den symbiotischen Mikroorganismen Saccharomyces und Acetobacter xylinum gewonnen. Ein erfindungsgemäß besonders bevorzugtes Kombucha-Produkt ist ein Produkt, das durch Fermentation von mit Saccharose gezuckertem Schwarztee mit den zwei symbiotischen Mikroorganismen Saccharomyces und Acetobacter xylinum gewonnen wurde und beispielsweise unter der INCI-Bezeichnung "Saccharomyces/Xylinum/Black Tea Ferment, Glycerin, Hydroxyethylcellulose" mit der Handelsbezeichnung "Kombuchka" als Glycerin-haltige verdickte Zubereitung von der Firma Sederma erhältlich ist.

Weitere erfindungsgemäß bevorzugte Wirkstoffe, die die Collagensynthese stimulieren, sind ausgewählt aus Olivenblattextrakten (Olea Europaea (Olive) Leaf Extract). Ein erfindungsgemäß besonders bevorzugter Olivenblattextrakt ist unter der Handelsbezeichnung Oleanoline DPG von der Firma Vincience erhältlich. Ein weiterer erfindungsgemäß besonders bevorzugter Olivenblattextrakt ist unter der Handelsbezeichnung Olea europ Fol extr. S. sicc. von der Firma Fruitarom erhältlich.

Olivenblattextrakte können einen hohen Gehalt an Oleanolsäure und/oder Oleanol aufweisen. Weitere erfindungsgemäß bevorzugte Wirkstoffe, die die Collagensynthese stimulieren, sind ausgewählt aus Oleanolsäure und Oleanol.

Weitere erfindungsgemäß bevorzugte Wirkstoffe, die die Collagensynthese stimulieren, sind ausgewählt aus Grüntee-Extrakten (Camellia Sinensis). Ein erfindungsgemäß besonders bevorzugter Grüntee-Extrakt ist liposomenverkapselter Grüntee-Extrakt, beispielsweise unter der Handelsbezeichnung Greenselect Phytosome von der Firma Indena erhältlich.

Weitere erfindungsgemäß bevorzugte kosmetische oder dermatologische Zusammensetzungen sind dadurch gekennzeichnet, dass sie zusätzlich zu der Zweierkombination aus mindestens einem Aporphin-Alkaloid und mindestens einem weiteren Wirkstoff, ausgewählt aus Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, sowie Mischungen dieser Wirkstoffe mindestens einen Wirkstoff, der die Collagensynthese stimuliert, in einer Gesamtmenge von 0,000001 - 5 Gew.-%, bevorzugt 0,00001 - 2 Gew.%, besonders bevorzugt 0,0001 - 1 Gew.-% und außerordentlich bevorzugt 0,005 - 0,5 Gew.-%, enthalten, jeweils bezogen auf den Gehalt an Aktivsubstanz in der gesamten Zusammensetzung.

Die Erscheinungen der Cellulite sind weitgehend unabhängig von der Hautalterung. Die mit der Hautalterung einhergehenden negativen Hautveränderungen verstärken jedoch das Erscheinungsbild der Cellulite weiter. Eine maßgebliche Folge der Hautalterung ist der Verlust an Collagen. Dieser basiert zum einen auf einer verminderten Collagensynthese in bestimmten Hautzellen, den Fibroblasten, zum anderen auf einem verstärkten Collagenabbau, insbesondere durch bestimmte Enzyme, wie der sogenannten Matrix-Metalloproteinase-1 (MMP-1). Insbesondere führt die UVinduzierte Expression der Matrix-Metalloproteinase-1 zu einem Abbau von Collagen-1, das ca. 85% des totalen Proteins in der extrazellulären Matrix ausmacht. Neben den Elementen des Zytoskeletts und den Gap Junction-Proteinen vermitteln weitere Proteine dem Hautgewebe mechanische Stabilität. Diese Proteine vermitteln eine Bindung zwischen Zellen und extrazellulärer Matrix (ECM). Hauptbestandteile der ECM sind Collagen und Hyaluronsäure. Für die Bindung von Zellen an die Proteine der ECM sind Rezeptoren verantwortlich, die auf der Zelloberfläche lokalisiert sind. So bindet z.B. der Oberflächenrezeptor CD44, dessen Expression beispielsweise durch Apfelkernextrakte gesteigert wird, an Hyaluronsäure.
Neuere Untersuchungen haben die Annahme erhärtet, dass durch den Collagenabbau das Integrin-vermittelte Wechselspiel zwischen Collagenmatrix und Fibroblasten gestört wird. Es wurde gezeigt, dass auf einer vorgeschädigten, kontrahierten und spannungsarmen Collagenmatrix die weitere Collagensynthese nur eingeschränkt erfolgt. Der Verlust an mechanischer Spannung scheint kausal an der verminderten Neusynthese von Pro-Collagen beteiligt zu sein (J. Varani et al., Reduced Fibroblast Interaction with Intact Collagen as a mechanism for depressed Collagen Synthesis in Photodamaged Skin, J. Invest. Dermatol. 122, 1471 -1479, 2004).

Weitere erfindungsgemäß bevorzugte kosmetische oder dermatologische Zusammensetzungen sind dadurch gekennzeichnet, dass sie zusätzlich zu der Zweierkombination aus mindestens einem Aporphin-Alkaloid und mindestens einem weiteren Wirkstoff, ausgewählt aus Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, sowie Mischungen dieser Wirkstoffe, mindestens einen weiteren, die Interaktion zwischen der extrazellulären Matrix und den Fibroblasten erhöhenden und/oder verbessernden Wirkstoff enthalten, der ausgewählt ist aus
- Apfelkernextrakten (Pyrus Malus (Apple) Fruit Extract),
- Lotuskeim-Extrakten (Nelumbo Nucifera Germ Extract),
- Extrakten aus Maiskörnern (Zea Mays (Corn) Kernel Extract),
- Rotweinextrakten,
- Traubenkernextrakten (Vitis Vinifera (Grape) Seed Extract), die bevorzugt aus der Chardonnay-Traube stammen,
- Extrakten aus Schwarzen Holunderblüten (Sambucus Nigra Flower Extract),
- Mischungen aus mindestens einem Extrakt aus Kakaobohnen (Theobroma cacao) und mindestens einem Extrakt aus den Blättern der Pfefferminze (Mentha piperita),
- Hydroxystilbenen und deren Estern, insbesondere Resveratrol und/oder Resveratrolmono-,
- -di- und -triphosphorsäureestern und deren Salzen, weiterhin aus Piceatannol, Piceatannolethern und Pinosylvin sowie Pinosylvinethern,
- Isoflavonoiden und Isoflavonoid-reichen Pflanzenextrakten,
- Dihydroquercetin (= Taxifolin),
- sowie Mischungen hiervon.

Weitere erfindungsgemäß bevorzugte Wirkstoffe, die die Interaktion zwischen der extrazellulären Matrix und den Fibroblasten erhöhen und/oder verbessern, sind ausgewählt aus Apfelkernextrakten (Pyrus Malus (Apple) Fruit Extract). Erfindungsgemäß besonders bevorzugte Apfelkernextrakte sind unter der Handelsbezeichnung Ederline von der Firma Seporga erhältlich. Das Produkt Ederline enthält Phytohormone, Isoflavonoide, Phytosterole, Triterpenoide, Tocopherole und natürliche Wachse. Ederline ist einmal in wasserlöslicher Form als Ederline-H (INCI: PEG-40 Hydrogenated Castor Oil, PPG-2-Ceteareth-9, Pyrus Malus (Apple) Fruit Extract), zum anderen in fettlöslicher Form als Ederline-L (INCI: Hexyldecanol, Pyrus Malus (Apple) Fruit Extract) erhältlich.

Erfindungsgemäß besonders bevorzugte kosmetische oder dermatologische Zusammensetzungen sind dadurch gekennzeichnet, dass sie den Rohstoff Ederline in Mengen von 0,1 - 10 Gew.-%, bevorzugt 1 - 8 Gew.-% und besonders bevorzugt 3 - 5 Gew.-%, jeweils bezogen auf die gesamte Zusammensetzung, enthalten. Erfindungsgemäß besonders bevorzugte kosmetische oder dermatologische Zusammensetzungen sind dadurch gekennzeichnet, dass sie mindestens einen Apfelkernextrakt in Mengen von 0,001 - 2 Gew.-%, bevorzugt 0,01 - 1,6 Gew.-% und besonders bevorzugt 0,03 - 1 Gew.-%, jeweils bezogen auf den Gehalt an Aktivsubstanz in der gesamten Zusammensetzung, enthalten.

Weitere erfindungsgemäß bevorzugte Wirkstoffe, die die Interaktion zwischen der extrazellulären Matrix und den Fibroblasten erhöhen und/oder verbessern, sind ausgewählt aus Lotuskeim-Extrakten. Ein erfindungsgemäß besonders bevorzugter Lotuskeim-Extrakt ist unter der Handelsbezeichnung Lotus Germ Extract mit der INCI-Bezeichnung Water, Butylene Glycol, Nelumbo Nucifera Germ Extract von der Firma Maruzen erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Extrakten aus Maiskörnern (Zea Mays (Corn) Kernel Extract). Ein erfindungsgemäß besonders bevorzugter Extrakt aus Maiskörnern ist unter der Handelsbezeichnung Deliner von der Firma Coletica erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Rotweinextrakten (Wine Extract). Ein erfindungsgemäß besonders bevorzugter Rotweinextrakt ist unter der Handelsbezeichnung Sepivinol R von der Firma Seppic erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Traubenkernextrakten (Vitis Vinifera (Grape) Seed Extract), die besonders bevorzugt aus der Chardonnay-Traube stammen. Erfindungsgemäß besonders bevorzugte Traubenkernextrakte sind unter der Handelsbezeichnung Herbalia Grape von der Firma Cognis oder unter der Handelsbezeichnung Crodarom Chardonnay von Croda erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Extrakten aus Schwarzen Holunderblüten (Sambucus Nigra Flower Extract). Ein erfindungsgemäß besonders bevorzugter Extrakt aus Schwarzen Holunderblüten ist unter der Handelsbezeichnung Sambucus AO von der Firma Alpaflor/ Centerchem bzw. von Permcos erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Mischungen aus mindestens einem Extrakt aus Kakaobohnen (Theobroma cacao) und mindestens einem Extrakt aus den Blättern der Pfefferminze (Mentha piperita). Erfindungsgemäß besonders bevorzugte Mischungen aus mindestens einem Extrakt aus Kakaobohnen (Theobroma cacao) und mindestens einem Extrakt aus den Blättern der Pfefferminze (Mentha piperita) - wobei wässrige, glycolische oder wässrig-glycolische Zubereitungen dieser Extraktmischungen besonders bevorzugt sind - sind unter den Handelsnamen Caomint, Caophenol, Caobromine, Caospice und Caoorange von Solabia erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Hydroxystilbenen und deren Estern, insbesondere Resveratrol (trans-Stilben-3,4'-5-triol) und/oder Resveratrolmono-, -di- und -triphosphorsäureestern und deren Salzen, weiterhin aus Piceatannol, Piceatannolethern und Pinosylvin sowie Pinosylvinethern. Ein erfindungsgemäß besonders bevorzugter Resveratrolphosphorsäureester ist Trisodium Resveratrol Triphosphate, z. B. erhältlich von Ajinomoto.

Weitere erfindungsgemäß bevorzugte Wirkstoffe, die die Interaktion zwischen der extrazellulären Matrix und den Fibroblasten erhöhen und/oder verbessern, sind ausgewählt aus Isoflavonoiden und Isoflavonoid-reichen Pflanzenextrakten.
Zu den Isofiavonoiden zählen erfindungsgemäß die Isoflavone und die Isofiavon-Glycoside.
Unter Isoflavonen sind erfindungsgemäß Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Besonders bevorzugte Isoflavone sind Daidzein, Genistein, Glycitein und Formononetin.
In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin.

Weiterhin ist es erfindungsgemäß bevorzugt, wenn die Isoflavone und/oder deren Glycoside als Bestandteile eines aus einer Pflanze gewonnenen Substanzgemisches, insbesondere eines pflanzlichen Extrakts, in den Zubereitungen enthalten sind. Solche pflanzlichen Substanzgemische können in dem Fachmann geläufiger Weise beispielsweise durch Auspressen oder Extrahieren aus Pflanzen wie Soja, insbesondere aus den Sojakeimen, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Lipobelle Soyaglycone (Mibelle AG Cosmetics), Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.
Erfindungsgemäß besonders bevorzugte kosmetische oder dermatologische Zusammensetzungen sind dadurch gekennzeichnet, dass sie mindestens ein Isoflavonoid in Gesamtmengen von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten kosmetischen Zusammensetzung, enthalten.

Ein weiterer erfindungsgemäß bevorzugter Wirkstoff, der die Interaktion zwischen der extrazellulären Matrix und den Fibroblasten erhöht und/oder verbessert, ist Dihydroquercetin (3,3',4',5,7-Pentahydroxyflavanon), das auch als Taxifolin bezeichnet wird.

Erfindungsgemäß besonders bevorzugte kosmetische oder dermatologische Zusammensetzungen sind dadurch gekennzeichnet, dass sie mindestens einen Wirkstoff, der die Interaktion zwischen der extrazellulären Matrix und den Fibroblasten erhöht und/oder verbessert, in einer Gesamtmenge von 0,000001 - 10 Gew.-%, bevorzugt 0,00001 - 5 Gew.-%, besonders bevorzugt 0,0001 - 2 Gew.-% und außerordentlich bevorzugt 0,005 - 0,5 oder 1 Gew.-%, enthalten, jeweils bezogen auf den Gehalt an Aktivsubstanz in der gesamten Zusammensetzung.

Weitere erfindungsgemäß bevorzugte kosmetische oder dermatologische Zusammensetzungen sind dadurch gekennzeichnet, dass sie zusätzlich zu der Zweierkombination aus mindestens einem Aporphin-Alkaloid und mindestens einem weiteren Wirkstoff, ausgewählt aus Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, sowie Mischungen dieser Wirkstoffe, mindestens einen weiteren Wirkstoff enthalten, der ausgewählt ist aus
- DNA- oder RNA-Oligonucleotiden,
- Vitaminen, Provitaminen und Vitaminvorstufen der Gruppen B, E, H und K und den Estern der vorgenannten Substanzen,
- α-Hydroxycarbonsäuren, α-Ketocarbonsäuren, β-Hydroxycarbonsäuren und deren Ester-, Lacton- und/oder Salzform,
- Flavonoiden und Flavonoid-reichen Pflanzenextrakten,
- Ubichinon und Ubichinol sowie deren Derivaten,
- Silymarin,
- Ectoin,
- anorganischen und organischen UV-Filtersubstanzen,
- selbstbräunenden,
- hautberuhigenden und
- sebumregulierenden Wirkstoffen sowie
- Mischungen dieser Wirkstoffe.

Weitere erfindungsgemäß bevorzugte kosmetische oder dermatologische Zusammensetzungen sind dadurch gekennzeichnet, dass sie zusätzlich zu der Zweierkombination aus mindestens einem Aporphin-Alkaloid und mindestens einem weiteren Wirkstoff, ausgewählt aus Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, sowie Mischungen dieser Wirkstoffe mindestens einen Wirkstoff enthalten, der ausgewählt ist aus DNA-Oligonucleotiden und RNA-Oligonucleotiden.
Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glycosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5 '-triphosphat) und GTP (Guanosin-5'-triphosphat).
Ein erfindungsgemäß besonders bevorzugtes Oligonucleotid ist das Thymidin-Dinucleotid.
In den besonders bevorzugten erfindungsgemäßen Zusammensetzungen sind die DNA-Oligonucleotide oder RNA-Oligonucleotide in Mengen von 0,00001 - 5 Gew.-%, bevorzugt 0,0001 - 1,0 Gew.-% und besonders bevorzugt 0,0005 - 0,5 Gew.-%, bezogen auf die gesamte Zusammensetzung, enthalten.

Weitere erfindungsgemäß bevorzugte kosmetische oder dermatologische Zusammensetzungen sind dadurch gekennzeichnet, dass sie zusätzlich zu der Zweierkombination aus mindestens einem Aporphin-Alkaloid und mindestens einem weiteren Wirkstoff, ausgewählt aus Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, sowie Mischungen dieser Wirkstoffe mindestens einen Wirkstoff enthalten, der ausgewählt ist aus mindestens einem Vitamin, Provitamin oder einer als Vitaminvorstufe bezeichnete Verbindung aus den Vitamingruppen B, E, H und K und den Estern der vorgenannten Substanzen.

Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf die gesamte Zusammensetzung, eingesetzt.
- Vitamin B₂, Trivialname Riboflavin, chemische Bezeichung 7,8-Dimethyl-10-(1-D-ribityl)-benzo[g]pteridin-2,4(3*H*,10*H*)-dion. Bevorzugt werden Riboflavin oder seine Derivate in Mengen von 0,05 bis 1 Gew.-%, bezogen auf die gesamte Zusammensetzung, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Zusammensetzungen bevorzugt in Mengen von 0,05 bis 1 Gew.-%, bezogen auf die gesamte Zusammensetzung, enthalten ist.
- Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (VIT-1) eingesetzt werden.

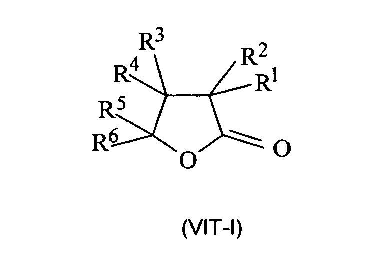

Bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (VITl) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.
Die genannten Verbindungen des Vitamin B₅-Typs sowie die 2-Furanonderivate sind in den erfindungsgemäßen Mitteln in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin B₆ ist in den erfindungsgemäßen Mitteln bevorzugt in Mengen von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-d]-imidazol-4-valeriansäure. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Zusammensetzungen sind dadurch gekennzeichnet, dass sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Zusammensetzung, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind dadurch gekennzeichnet, dass sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Zusammensetzung, enthalten.

Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.-%, bezogen auf die gesamte Zusammensetzung, enthalten.
Vitamin H ist eine andere Bezeichnung für Biotin oder Vitamin B₇ (siehe oben).
Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K ist bevorzugt in Mengen von 0,0001 bis 1,0 Gew.-%, insbesondere 0,01 bis 0,5 Gew.-%, jeweils bezogen auf die gesamte Zusammensetzung, enthalten.

Erfindungsgemäß besonders bevorzugte kosmetische oder dermatologische Zusammensetzungen sind dadurch gekennzeichnet, dass sie mindestens eine der folgenden Wirkstoffkombinationen enthalten:
1,2,9,10-Tetramethoxyaporphin und Folsäure, denn es wurde überraschend festgestellt, dass diese Kombination die Mikrozirkulation in den Zellen der Haut verbessert und dadurch die Festigkeit der Haut verbessert,
1,2,9,10-Tetramethoxyaporphin und 1,3,7-Trimethylxanthin und Folsäure, denn es wurde überraschend festgestellt, dass diese Kombination die Mikrozirkulation in den Zellen der Haut weiter steigern und dadurch die Festigkeit der Haut in überraschend hohem Maße verbessern kann,
1,2,9,10-Tetramethoxyaporphin und Folsäure und N-Palmitoyl-Gly-His-Lys und N-Palmitoyl-Gly-Gln-Pro-Arg, denn es wurde überraschend festgestellt, dass diese Kombination die Mikrozirkulation in den Zellen der Haut weiter steigern und dadurch die Festigkeit der Haut in unerwartet hohem Maße verbessern kann,
1,2,9,10-Tetramethoxyaporphin und Folsäure und N-Palmitoyl-Lys-Thr-Thr-Lys-Ser, denn es wurde überraschend festgestellt, dass diese Kombination die Mikrozirkulation in den Zellen der Haut verbessert und dadurch die Festigkeit der Haut verbessert,
1,2,9,10-Tetramethoxyaporphin und Carnitin und Folsäure, denn es wurde überraschend festgestellt, dass diese Kombination die Lipolyse in der Subcutis verbessert und dadurch die Festigkeit der Haut verbessert.

Weitere erfindungsgemäß bevorzugte kosmetische oder dermatologische Zusammensetzungen sind dadurch gekennzeichnet, dass sie zusätzlich zu der Zweierkombination aus mindestens einem Aporphin-Alkaloid und mindestens einem weiteren Wirkstoff, ausgewählt aus Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, sowie Mischungen dieser Wirkstoffe mindestens einen Wirkstoff enthalten, der ausgewählt ist aus mindestens einer α-Hydroxycarbonsäure, α-Ketocarbonsäure oder β-Hydroxycarbonsäure oder deren Ester-, Lacton- und/ oder Salzform. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Eine besonders bevorzugte α-Ketocarbonsäure ist Brenztraubensäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Weitere Derivate der vorgenannten Säuren sind deren physiologisch verträglichen Salze, bevorzugt die Zink-, Kupfer- und Mangansalze, die Salze der Alkali- und der Erdalkalimetalle und die Ammonium-, Alkylammonium-, Alkanolammonium- und Glucammoniumsalze, besonders bevorzugt die Natrium-, Kalium-, Magnesium- und Calcium-Salze.
Die α-Hydroxycarbonsäuren, α-Ketocarbonsäuren oder β-Hydroxycarbonsäuren oder ihre Derivate sind bevorzugt in einer Gesamtmenge von 0,1 - 10 Gew.-%, besonders bevorzugt 0,5 - 5 Gew.-% und außerordentlich bevorzugt 1 - 2 Gew.-%, jeweils bezogen auf die gesamte Zusammensetzung, enthalten.

Weitere erfindungsgemäß bevorzugte kosmetische oder dermatologische Zusammensetzungen sind dadurch gekennzeichnet, dass sie zusätzlich zu der Zweierkombination aus mindestens einem Aporphin-Alkaloid und mindestens einem weiteren Wirkstoff, ausgewählt aus Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, sowie Mischungen dieser Wirkstoffe mindestens einen Wirkstoff enthalten, der ausgewählt ist aus mindestens einem Flavonoid und/oder mindestens einem Flavonoid-reichen Pflanzenextrakt.
Die erfindungsgemäß bevorzugten Flavonoide umfassen die Glycoside der Flavone, der 3-Hydroxyflavone (Flavonole) und der Aurone. Die Flavanone und die Isoflavone sind erfindungsgemäß ausdrücklich von den Flavonoiden ausgenommen. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)flavon-3-(6-O-(6-deoxy-α-L-mannopyranosyl)-β-D-glucopyranosid)), Eriodictin und Apigenin-7-glucosid (4',5,7-Trihydroxyflavon-7-glucosid). Erfindungsgemäß außerordentlich bevorzugte Flavonoide sind α-Glucosylrutin, Naringin und Apigenin-7-glucosid.
Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin und Neohesperidindihydrochalkon.
Erfindungsgemäß werden die Flavonoide in Mengen von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, eingesetzt.

Weitere erfindungsgemäß bevorzugte kosmetische oder dermatologische Zusammensetzungen sind dadurch gekennzeichnet, dass sie zusätzlich zu der Zweierkombination aus mindestens einem Aporphin-Alkaloid und mindestens einem weiteren Wirkstoff, ausgewählt aus Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, sowie Mischungen dieser Wirkstoffe mindestens einen Wirkstoff enthalten, der ausgewählt ist aus mindestens einem Ubichinon oder einem Ubichinol oder deren Derivaten. Ubichinole sind die reduzierte Form der Ubichinone. Die erfindungsgemäß bevorzugten Ubichinone weisen die Formel (UBI-I) auf: 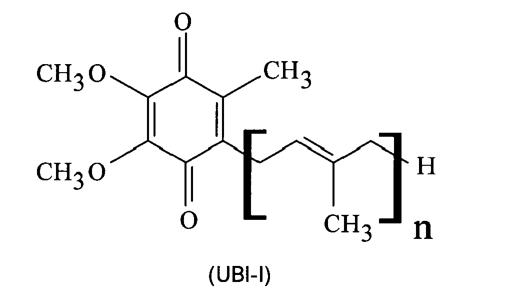 mit n = 6, 7, 8, 9 oder 10.
Besonders bevorzugt ist das Ubichinon der Formel (UBI-I) mit n = 10, auch bekannt als Coenzym Q10.
Erfindungsgemäß werden die Ubichinone, Ubichinole oder deren Derivate in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, eingesetzt.

Erfindungsgemäß besonders bevorzugte kosmetische oder dermatologische Zusammensetzungen sind dadurch gekennzeichnet, dass sie mindestens eine der folgenden Wirkstoffkombinationen enthalten:
1,2,9,10-Tetramethoxyaporphin und Coenzym Q 10, denn es wurde überraschend festgestellt, dass diese Kombination die Mikrozirkulation in den Zellen der Haut verbessert und dadurch die Festigkeit der Haut verbessert,
1,2,9,10-Tetramethoxyaporphin und 1,3,7-Trimethylxanthin und Coenzym Q 10, denn es wurde überraschend festgestellt, dass diese Kombination die Mikrozirkulation in den Zellen der Haut weiter steigern und dadurch die Festigkeit der Haut in überraschend hohem Maße verbessern kann,
1,2,9,10-Tetramethoxyaporphin und Carnitin und Coenzym Q 10, denn es wurde überraschend festgestellt, dass diese Kombination die Mikrozirkulation in den Zellen der Haut weiter steigern und dadurch die Festigkeit der Haut in unerwartet hohem Maße verbessern kann.

Weitere erfindungsgemäß bevorzugte kosmetische oder dermatologische Zusammensetzungen sind dadurch gekennzeichnet, dass sie zusätzlich zu der Zweierkombination aus mindestens einem Aporphin-Alkaloid und mindestens einem weiteren Wirkstoff, ausgewählt aus Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, sowie Mischungen dieser Wirkstoffe weiterhin Silymarin enthalten. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören.
Erfindungsgemäß wird Silymarin in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, eingesetzt.

Weitere erfindungsgemäß bevorzugte kosmetische oder dermatologische Zusammensetzungen sind dadurch gekennzeichnet, dass sie zusätzlich zu der Zweierkombination aus mindestens einem Aporphin-Alkaloid und mindestens einem weiteren Wirkstoff, ausgewählt aus Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, sowie Mischungen dieser Wirkstoffe weiterhin Ectoin enthalten. Ectoin ist der Trivialname für 2-Methyl-1,4,5,6-tetrahydropyrimidin-4-carboxylat. Erfindungsgemäß ist Ectoin bevorzugt in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

Erfindungsgemäß besonders bevorzugte kosmetische oder dermatologische Zusammensetzungen sind dadurch gekennzeichnet, dass sie mindestens eine der folgenden Wirkstoffkombinationen enthalten:
1,2,9,10-Tetramethoxyaporphin und Ectoin, denn es wurde überraschend festgestellt, dass diese Kombination die Mikrozirkulation in den Zellen der Haut verbessert und dadurch die Festigkeit der Haut verbessert,
1,2,9,10-Tetramethoxyaporphin und 1,3,7-Trimethylxanthin und Ectoin, denn es wurde überraschend festgestellt, dass diese Kombination die Mikrozirkulation in den Zellen der Haut weiter steigern und dadurch die Festigkeit der Haut in überraschend hohem Maße verbessern kann.

Weitere erfindungsgemäß bevorzugte kosmetische oder dermatologische Zusammensetzungen sind dadurch gekennzeichnet, dass sie zusätzlich zu der Zweierkombination aus mindestens einem Aporphin-Alkaloid und mindestens einem weiteren Wirkstoff, ausgewählt aus Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, sowie Mischungen dieser Wirkstoffe weiterhin mindestens eine anorganische und/oder mindestens eine organische UV-Filtersubstanz enthalten.
Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filtermischungen ist erfindungsgemäß bevorzugt.
Die erfindungsgemäß bevorzugten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Die Benzoxazol-Derivate liegen vorteilhaft in gelöster Form in den erfindungsgemäß bevorzugten kosmetischen Zubereitungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen. Erfindungsgemäß besonders bevorzugte öllösliche UV-Filter sind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 2-(4'-Diethylamino-2'-hydroxybenzoyl)-benzoesäurehexylester (auch: Aminobenzophenon, unter der Bezeichnung Uvinul A Plus bei der Firma BASF erhältlich), 4-Methoxybenzmalonsäuredi-2-ethylhexylester, an Polymere gebundene UV-Filter, z. B. das 3-(4-(2,2-Bis-Ethoxycarbonylvinyl)-phenoxy)propenyl)-methoxysiloxan/ Dimethylsiloxan-Copolymer mit der INCI-Bezeichnung Dimethicodiethylbenzal malonate (CAS-Nr. 207574-74-1, Parsol^{®} SLX), Triazinderivate, wie z. B. 2,4-Bis-{[4-(2-Ethyl-hexyloxy)-2-hydroxy]-phenyl}-6-(4-methoxyphenyl)-1,3,5-triazin (INCI: Bis-Ethylhexyloxyphenol Methoxyphenyl Triazin, unter dem Namen Tinosorb S bei CIBA erhältlich), Dioctylbutylamidotriazon (INCI: Diethylhexyl Butamido Triazone, unter dem Namen Uvasorb^{®} HEB bei Sigma 3V erhältlich), 2,4,6-Trianilino-(p-carbo-2'-ethyl-1'-hexyloxy)-1,3,5-triazin (Ethylhexyl Triazone, Uvinul^{®} T 150), 2-[4,6-Bis(2,4-dimethylphenyl)-1,3,5-triazin-2-yl]-5-(octyloxy)phenol (CAS Nr.: 2725-22-6), 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma), die Benzotriazolderivate 2,2'-Methylen-bis-(6-(2H-benzotriazol-2-yl)-4-(1,1,3,3-tetramethylbutyl)-phenol) [Tinosorb M (Ciba)], 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4), 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane, 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin oder auch Aniso Triazin, erhältlich als Tinosorb^{®} S von CIBA), 2,4-Bis-{[4-(3-sulfonato)-2-hydroxy-propyloxy)-2-hydroxy]-phenyl}-6-(4-methoxyphenyl)-1,3,5-triazin-Natriumsalz, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-[4-(2-methoxyethylcarboxyl)-phenylamino]-1,3,5-triazin, 2,4-Bis-{[4-(3-(2-propyloxy)-2-hydroxy-propyloxy)-2-hydroxy]-phenyl}-6-[4-(ethylcarboxyl)-phenylamino]-1,3,5-triazin, 2,4-Bis-{[4-(2-ethylhexyloxy)-2-hydroxy]-phenyl}-6-(1-methyl-pyrrol-2-yl)-1,3,5-triazin, 2,4-Bis-{[4-tris(trimethylsiloxy-silylpropyloxy)-2-hydroxy]-phenyl}-6-(4-methoxyphenyl)-1,3,5-triazin, 2,4-Bis-{[4-(2-methylpropenyloxy)-2-hydroxy]-phenyl}-6-(4-methoxyphenyl)-1,3,5-triazin, 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin sowie Mischungen der genannten Komponenten.

Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, insbesondere die Sulfonsäure selbst mit der INCI Bezeichnung Phenylbenzimidazole Sulfonic Acid (CAS.-Nr. 27503-81-7), die beispielsweise unter dem Handelsnamen Eusolex 232 bei Merck oder unter Neo Heliopan Hydro bei Symrise erhältlich ist, und das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonate (CAS-Nr.: 180898-37-7), das beispielsweise unter dem Handelsnamen Neo Heliopan AP bei Symrise erhältlich ist, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze mit der INCI-Bezeichnung Terephthalydene Dicampher Sulfonic Acid (CAS.-Nr.: 90457-82-2, als Mexoryl SX von der Firma Chimex erhältlich).
Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'-methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.
Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Das Coatingmaterial kann aber in einer anderen bevorzugten Ausführungsform auch anorganische Materialien umfassen; besonders bevorzugt ist Silicoiumdioxid als Coatingmaterial. Besonders bevorzugte Pigmentmaterialien sind Titandioxid und Zinkoxid.
Erfindungsgemäß sind die organischen UV-Filtersubstanzen bevorzugt in Mengen von 0,1 - 30 Gew.-%, besonders bevorzugt 0,5 - 20 Gew.-%, außerordentlich bevorzugt 1,0 - 15 Gew.-% und weiter bevorzugt 3,0 - 10 Gew.-%, jeweils bezogen auf die gesamte Zusammensetzung, enthalten. Erfindungsgemäß sind die anorganischen UV-Filtersubstanzen bevorzugt in Mengen von 0,1 - 15 Gew.-%, besonders bevorzugt 0,5 - 10 Gew.-%, außerordentlich bevorzugt 1,0 - 5 Gew.-% und weiter bevorzugt 2,0 - 4,0 Gew.-%, jeweils bezogen auf die gesamte Zusammensetzung, enthalten.

Weitere erfindungsgemäß bevorzugte kosmetische oder dermatologische Zusammensetzungen sind dadurch gekennzeichnet, dass sie zusätzlich zu der Zweierkombination aus mindestens einem Aporphin-Alkaloid und mindestens einem weiteren Wirkstoff, ausgewählt aus Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, sowie Mischungen dieser Wirkstoffe weiterhin mindestens einen selbstbräunenden Wirkstoff enthalten. Erfindungsgemäß bevorzugte selbstbräunende Wirkstoffe sind ausgewählt aus Dihydroxyaceton, Erythrulose und 5,6-Dihydroxyindolin.

Erfindungsgemäß sind die selbstbräunenden Wirkstoffe in einer Gesamtmenge von 0,01 - 15 Gew.-%, bevorzugt 0,1 - 10 Gew.-%, besonders bevorzugt 1,0 - 5 Gew.-% und außerordentlich bevorzugt 2,0 - 4,0 Gew.-%, jeweils bezogen auf die gesamte Zusammensetzung, enthalten.

Weitere erfindungsgemäß bevorzugte kosmetische oder dermatologische Zusammensetzungen sind dadurch gekennzeichnet, dass sie zusätzlich zu der Zweierkombination aus mindestens einem Aporphin-Alkaloid und mindestens einem weiteren Wirkstoff, ausgewählt aus Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, sowie Mischungen dieser Wirkstoffe weiterhin mindestens einen hautberuhigenden Wirkstoff enthalten. Erfindungsgemäß bevorzugte hautberuhigende Wirkstoffe sind ausgewählt aus Allantoin, α-Bisabolol, α-Liponsäure, Extrakten aus Centella asiatica, beispielsweise erhältlich unter der Bezeichnung Madecassicoside von DSM, Glycyrrethinsäure, die besonders bevorzugt in Liposomen verkapselt vorliegt und in dieser Form z. B. unter dem Handelsnamen Calmsphere von Soliance erhältlich ist, Mischungen aus Getreidewachsen, Extrakten aus Schibutter und Argania Spinosa-ÖI mit der INCI-Bezeichnung "Spent grain wax and Butyrospermum Parkii (shea butter) extract and Argania Spinosa Kernel Oil", wie sie z. B. unter der Handelsbezeichnung Stimu-Tex AS von der Firma Pentapharm erhältlich sind, Extrakten aus Vanilla Tahitensis, wie sie z. B. unter der Handelsbezeichnung Vanirea (INCI : Vanilla Tahitensis Fruit Extract) von der Firma Solabia erhältlich sind, Alginhydrolysaten, wie sie z. B. unter der Handelsbezeichnung Phycosaccharide, insbesondere Phycosaccharide Al, von der Firma Codif erhältlich sind, Extrakten aus Bacopa Monniera, wie sie z. B. unter der Handelsbezeichnung Bacocalmine von der Firma Sederma erhältlich sind, Extrakten aus der Rooibos-Pflanze, wie sie z. B. unter dem Handelsnamen Rooibos Herbasec MPE von der Firma Cosmetochem erhältlich sind, Hefeextrakten, besonders bevorzugt das Handelsprodukt Drieline (INCI-Bezeichnung "Sorbitol, Yeast Extract"), erhältlich von der Firma Lanatech, den physiologisch verträglichen Salzen von Sterolsulfaten, wie sie z. B. unter dem Handelsnamen Phytocohesine (INCI: Sodium Beta-Sitosterylsulfate) von der Firma Vincience erhältlich sind, Aminodicarbonsäuren mit einer C-Kettenlänge von 3 - 6 Kohlenstoffatomen sowie deren physiologisch verträglichen Salzen, bevorzugt ausgewählt aus Aminomalonsäure, Aminobernsteinsäure (= Asparaginsäure), Aminoglutarsäure und Aminoadipinsäure sowie deren physiologisch verträglichen Salzen wie Kaliumaspartat und Magnesiumaspartat, Extrakten aus den Samen von Cucurbita pepo (Zucchini), Extrakten aus Mentha piperita, insbesondere aus den Blättern von Mentha piperita, N-acylierten Alkanolaminen, insbesondere N-Palmitoylethanolamin, Hydrolysaten und/oder Extrakten aus der Alge *Enteromorpha compressa* (Darmtang, eine Grünalge), insbesondere aus dem Vegetationskörper (Thallus) der Alge, kommerziell erhältlich z. B. unter dem Handelsnamen Enteline 2, weiterhin ausgewählt aus dem Dipeptid Tyr-Arg, dessen Estern und dessen N-Acyl-Derivaten, insbesondere N-Acetyl-Tyr-Arg-hexyldecylester (z. B. Calmosensine von Sederma, sowie beliebigen Mischungen dieser Substanzen.

Erfindungsgemäß besonders bevorzugte Extrakte aus den Samen von Cucurbita pepo (Zucchini) sind beispielsweise in den Handelsprodukten Curbilene (ex Indena SpA, INCI: Cucurbita pepo (pumpkin) seed extract), Ocaline (ex Soliance, INCI: Sea Water (and) Water (and) Cucurbita pepo (pumpkin) seed extract), ARP 100 (ex Greentech, INCI: Water, Alcohol, Serenoa Serrulata Fruit Extract, Epilobium Angustifolium Extract, Cucurbita Pepo (Pumpkin) Seed Extract), ARP 100 Huileux (ex Greentech, INCI: Caprylic/Capric Triglyceride, Serenoa Serrulata Fruit Extract, Epilobium Angustifolium Flower/Leaf/Stem Extract, Cucurbita Pepo (Pumpkin) Seed Extract), Cucurbitine (ex Christian Dior Parfums, INCI: Water (and) Cucurbita pepo (pumpkin) seed extract), Herbasol Extract Pumpkin (ex Cosmetochem, INCI: Water (and) Propylene glycol (and) Cucurbita pepo (pumpkin) seed extract) und Pumpkin Extract (ex Draco Natural Products, INCI: Water (and) Cucurbita pepo (pumpkin) seed extract) enthalten.
Erfindungsgemäß besonders bevorzugte Extrakte aus Mentha piperita, insbesondere aus den Blättern von Mentha piperita, sind beispielsweise in den Handelsprodukten Calmiskin OP (ex Silab, INCI: Water (and) Mentha piperita (Peppermint) Leaf Extract) und Caomint (ex Solabia, INCI: Propylene Glycol, Water, Mentha piperita (Peppermint) Leaf Extract, Theobroma Cacao (Cocoa) Extract) enthalten.
Die hautberuhigenden Wirkstoffe sind bevorzugt in Mengen von 0,001 bis 5 Gew.-%, besonders bevorzugt 0,01 bis 2 Gew.-% und außerordentlich bevorzugt 0,1 bis 1 Gew.-%, jeweils bezogen auf die gesamte Zusammensetzung, enthalten.

Weitere erfindungsgemäß bevorzugte kosmetische oder dermatologische Zusammensetzungen sind dadurch gekennzeichnet, dass sie zusätzlich zu der Zweierkombination aus mindestens einem Aporphin-Alkaloid und mindestens einem weiteren Wirkstoff, ausgewählt aus Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, sowie Mischungen dieser Wirkstoffe weiterhin mindestens einen sebumregulierenden Wirkstoff. Erfindungsgemäß bevorzugte sebumregulierende Wirkstoffe sind ausgewählt aus Azelainsäure, Sebacinsäure, 10-Hydroxydecansäure, 1,10-Decandiol, die als erfindungsgemäß besonders bevorzugte Dreierkombination in dem Handelsprodukt Acnacidol PG von Vincience eingesetzt werden, weiterhin aus dem Handelsprodukt Azeloglicina (Potassium Azeloyl Diglycinate) von Sinerga, Extrakten aus Spiraea Ulmaria, wie sie z. B. im Produkt Seboregul der Firma Silab enthalten sind, weiterhin aus wasser- und öllöslichen Extrakten aus Hamamelis, Klettenwurzel und Brennessel, Zimtbaumextract (z. B. Sepicontrol^{®} A5 von Seppic), Chrysanthemenextrakt (z. B. Laricyl^{®} von Laboratoires Sérobiologiques) und den Wirkstoffmischungen Asebiol^{®} BT 2 (Laboratoires Sérobiologiques, INCI: Aqua, Hydrolyzed Yeast Protein, Pyridoxine, Niacinamide, Glycerin, Panthenol, , Allantoin, Biotin) und Antifettfaktor^{®} COS-218/2-A (Cosmetochem, INCI: Aqua, Cetyl-PCA, PEG-8 Isolauryl Thioether, PCA, Cetyl Alcohol).

Die sebumregulierenden Wirkstoffe sind bevorzugt in einer Gesamtmenge von 0,001 bis 5 Gew.-%, besonders bevorzugt 0,01 bis 2 Gew.% und außerordentlich bevorzugt 0,1 bis 1 Gew.-%, jeweils bezogen auf die gesamte Zusammensetzung, enthalten.

Ein weiterer Gegenstand der vorliegenden Erfindung ist die nicht-therapeutische, kosmetische Verwendung einer kosmetischen oder dermatologischen Zusammensetzung gemäß einem der Ansprüche 1 - 20 zur Anti-Cellulite-Behandlung der Haut und/oder zur Steigerung der Hautfestigkeit und/oder zur Verbesserung der mechanischen Stabilität der Haut und/oder zur Steigerung der Hautelastizität und/oder der Hautstraffheit und/oder zur Glättung der Haut und/oder zur Verbesserung des optischen Erscheinungsbildes der Haut und/oder zur Anregung der Mikrozirkulation in der Haut und/oder zur Unterstützung der Lipolyse in der Subcutis und/oder zur Inhibierung der Lipogenese in der Subcutis.
Die Hautfestigkeit und die mechanische Stabilität der Haut kann zum einen z. B. über den Torsionswinkel, der mit dem Dermal Torque Meter gemessen werden kann, zum anderen über den Parameter U_{F} (totale Ausdehnbarkeit der Haut bei Unterdruck), der mit dem Cutometer gemessen werden kann, bestimmt werden.

Ein weiterer Gegenstand der vorliegenden Erfindung ist die Verwendung einer Wirkstoffkombination aus mindestens einem Aporphin-Alkaloid und mindestens einem weiteren Wirkstoff, ausgewählt aus
a) Purin und Purin-Derivaten,
b) natürlichen Betainverbindungen,
c) Harnstoff und alkyl- oder hydroxyalkylsubstituierten Harnstoffen,
d) Monomeren, Oligomeren und Polymeren von Aminosäuren, N-C₂-C₂₄-Acylaminosäuren und/oder den Estern und/oder den physiologisch verträglichen Salzen dieser Substanzen,
e) Polysacchariden,
f) hyperämisierenden Wirkstoffen,
g) sowie Mischungen dieser Wirkstoffe,
zur Herstellung einer kosmetischen oder dermatologischen Zusammensetzung
- zur Behandlung der Cellulite und/oder
- zur Steigerung der Hautfestigkeit und/oder
- zur Verbesserung der mechanischen Stabilität der Haut und/oder
- zur Steigerung der Hautelastizität und/oder
- zur Straffung der Haut und/oder
- zur Glättung der Haut und/oder
- zur Verbesserung des optischen Erscheinungsbildes der Haut
- zur Anregung der Mikrozirkulation in der Haut und/oder
- zur Unterstützung der Lipolyse in der Subcutis und/oder
- zur Inhibierung der Lipogenese in der Subcutis.

Weitere erfindungsgemäß bevorzugte kosmetische oder dermatologische Zusammensetzungen sind dadurch gekennzeichnet, dass sie zusätzlich zu der Zweierkombination aus mindestens einem Aporphin-Alkaloid und mindestens einem weiteren Wirkstoff, ausgewählt aus Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, sowie Mischungen dieser Wirkstoffe weiterhin mindestens einen konditionierenden Wirkstoff enthalten. Unter konditionierenden Wirkstoffen sind erfindungsgemäß solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen.
Erfindungsgemäß bevorzugte konditionierende Wirkstoffe sind ausgewählt aus Fettstoffen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-alkylethern mit insgesamt 12 bis 36 C-Atomen, z. B. Di-n-octylether und n-Hexyl-n-octylether, Fettsäuren, besonders linearen und/oder verzweigten, gesättigten und/oder ungesättigten C₈₋₃₀-Fettsäuren, Fettalkoholen, besonders gesättigten, ein- oder mehrfach ungesättigten, verzweigten oder unverzweigten Fettalkoholen mit 4 - 30 Kohlenstoffatomen, die mit 1 - 75, bevorzugt 5 - 20 Ethylenoxid-Einheiten ethoxyliert und/oder mit 3 - 30, bevorzugt 9 - 14 Propylenoxid-Einheiten propoxyliert sein können, Esterölen, das heißt Estern von C₆₋₃₀-Fettsäuren mit C₂₋₃₀-Fettalkoholen, Hydroxycarbonsäurealkylestern, Dicarbonsäureestern wie Di-n-butyladipat sowie Diolestern wie Ethylenglykoldioleat oder Propylenglykoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol^{®} CC), Mono,- Di- und Trifettsäureestern von gesättigten und/oder ungesättigten linearen und/oder verzweigten Fettsäuren mit Glycerin, die mit 1 - 10, bevorzugt 7 - 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter C₁₆₋₃₀-Fettsäuren, z. B. gehärteten Triglyceridfetten, Phospholipiden, beispielsweise Sojalecithin, Ei-Lecithin und Kephalinen, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/oder HydroxyGruppen enthalten, bevorzugt Substanzen mit den INCI-Bezeichnungen Dimethiconol, Amodimethicone oder Trimethylsilylamodimethicone.
Die Einsatzmenge der Fettstoffe beträgt bevorzugt 0,1 - 99 Gew.-%, besonders bevorzugt 2 - 50 Gew.-% und außerordentlich bevorzugt 5 - 20 Gew.-%, jeweils bezogen auf das gesamte Hautbehandlungsmittel.

Bevorzugt liegen die erfindungsgemäßen kosmetischen oder dermatologischen Zusammensetzungen in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.
In einer erfindungsgemäß besonders bevorzugten Ausführungsform liegen die Mittel als Mikroemulsion vor. Unter Mikroemulsionen werden im Rahmen der Erfindung neben den thermodynamisch stabilen Mikroemulsionen auch die sogenannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl-Emulsionen umwandeln. Beim anschließenden Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.
In der bevorzugten Ausführungsform als Emulsion enthalten die erfindungsgemäßen Zusammensetzungen mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole, C₁₂-C₂₂-Fettsäuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov^{®} 68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsäureester von Zuckern und Zuckeralkoholen wie Sorbit, Polyglycerine und Polyglycerinderivate, bevorzugt Polyglyceryl-2-dipolyhydroxystearat (Handelsprodukt Dehymuls^{®} PGPH) und Polyglyceryl-3-diisostearat (Handelsprodukt Lameform^{®} TGI) sowie lineare und verzweigte C₈-C₃₀-Fettsäuren und deren Na-, K-, Ammonium-, Ca-, Mg- und Zn - Salze.
Die erfindungsgemäßen Zusammensetzungen enthalten die Emulgatoren bevorzugt in Mengen von 0,1 bis 25 Gew.-%, besonders bevorzugt 0,5 - 15 Gew.-%, bezogen auf die gesamte Zusammensetzung.
In einer besonders bevorzugten Ausführungsform ist mindestens ein nichtionischer Emulgator mit einem HLB-Wert von 8 und darunter enthalten. Derartige bevorzugte Emulgatoren sind insbesondere Verbindungen der allgemeinen Formel R¹ - O - R², in der R¹ eine primäre lineare Alkyl-, Alkenyl- oder Acylgruppe mit 20 - 30 C-Atomen, bevorzugt ein Behenyl-Rest, und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen, bevorzugt Wasserstoff, darstellt. Eine besonders bevorzugte Verbindung R¹ - O - R² ist Behenylalkohol. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythritmonoerucat. Die Anwesenheit von Emulgatoren der allgemeinen Formel R¹ - O - R² kann zu lamellaren Strukturen in der Emulsion führen, die besonders günstige Effekte auf die Wiederherstellung der lamellaren Struktur der Haut haben können. Besonders bevorzugte erfindungsgemäße Zusammensetzungen sind daher dadurch gekennzeichnet, dass sie in Form einer lamellare Strukturen aufweisenden Öl-in-Wasser-Emulsion vorliegen.
In einer weiteren besonders bevorzugten Ausführungsform liegen die erfindungsgemäßen Zusammensetzungen in Form einer Wasser-in-Öl-Emulsion vor. Derartige Wasser-in-ÖI-Emulsionen können so formuliert werden, dass die eine höhere Viskosität aufweisen, so dass sie sorgfältig in die Haut einzumassieren sind. Dies unterstützt die Anticellulite-Wirkung der erfindungsgemäßen Zusammensetzungen. Genau so gut lassen sich aber auch niedrig-viskose Wasser-in-ÖI-Emulsionen formulieren, insbesondere auf Basis des polymeren Wasser-in-ÖI-Emulgators PEG-30 Dipolyhydroxystearat, erhältlich z. B. unter dem Handelsnamen Arlacel P 135 von Uniqema. Insbesondere mit Hilfe dieses Emulgators lassen sich sprühbare Wasser-in-ÖI-Emulsionen formulieren.
Weitere geeignete Zusatzstoffe sind Verdickungsmittel, z. B. natürliche und synthetische Tone und Schichtsilikate wie Bentonit, Hectorit, Montmorillonit oder Laponite^{®}, oder anionische Polymere aus Acrylsäure, Methacrylsäure, Crotonsäure, Maleinsäureanhydrid und 2-Acrylamido-2-methylpropan-sulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®}600, Simulgel^{®} NS und Simulgel^{®} EG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).
Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden.
Weitere bevorzugte Zusatzstoffe sind Antioxidantien, Konservierungsmittel, Lösungsmittel wie Ethanol, Isopropanol, Ethylenglykol, Propylenglykol, Propylenglykolmonoethylether, Glycerin und Diethylenglykol, Adsorbentien und Füllstoffe, wie Talkum und Veegum^{®}, Parfümöle, Pigmente sowie Farbstoffe zum Anfärben des Mittels, Substanzen zur Einstellung des pH-Wertes, Komplexbildner wie EDTA, NTA, β-Alanindiessigsäure und Phosphonsäuren, Treibmittel wie Propan-Butan-Gemische, Pentan, Isopentan, Isobutan, N₂O, Dimethylether, CO₂ und Luft.

Da die erfindungsgemäßen Zusammensetzungen insbesondere für die (großflächige) Anwendung auf der Körperhaut bestimmt sind, ist es besonders vorteilhaft und praktisch, wenn sie als sprühbare Zusammensetzung vorliegen. Ein weiterer Gegenstand der vorliegenden Erfindung ist daher eine Zusammensetzung gemäß einem der Ansprüche 1 - 19, die in einem Sprühspender oder Pumpspender verpackt ist.

### Ausführungsbeispiele

Die nachfolgenden Formulierungsbeispiele sollen den Gegenstand der Erfindung erläutern, ohne ihn hierauf zu beschränken.

Alle Mengenangaben sind in Gew.-%, bezogen auf die gesamte Zusammensetzung.

### 1. Oel-in-Wasser-Emulsionen

### 1. Hautcremes

Die Zusammensetzungen 1.1 - 1.4 sind bevorzugte Ausführungsformen von pflegenden Tagescremes mit einem Anticellulite-Effekt bzw. einem Effekt zur Verbesserung der Silhouette des Gesichts, insbesondere im Kinn-Bereich, mit erfindungsgemäß verbesserter Wirkung.

| | 1.1 | 1.2 | 1.3 | 1.4 |
|---|---|---|---|---|
| Distelöl | 3,00 | 3,00 | 3,00 | 3,00 |
| Myritol 318 | 5,00 | 5,00 | 5,00 | 5,00 |
| Novata AB | 2,00 | 2,00 | 2,00 | 2,00 |
| Behenyl Alcohol | 1,00 | 1,00 | 1,00 | 1,00 |
| Cutina MD | 2,00 | 2,00 | 2,00 | 2,00 |
| Cetearyl Alcohol | 1,00 | 1,00 | 1,00 | 1,00 |
| Isopropylstearat | 4,00 | 4,00 | 4,00 | 4,00 |
| Shea Butter | 2,00 | 2,00 | 2,00 | 2,00 |
| Baysilone-Öl M 350 | 1,00 | 1,00 | 1,00 | 1,00 |
| Controx KS | 0,05 | 0,05 | 0,05 | 0,05 |
| Propylparaben | 0,20 | 0,20 | 0,20 | 0,20 |
| Dow Corning 1501 Fluid | 1,00 | 1,00 | 1,00 | 1,00 |
| Dry Flo Plus | 1,00 | 1,00 | 1,00 | 1,00 |
| TiO₂ | 0,50 | 0,50 | 0,50 | 0,50 |
| Hexandiol | 6,00 | 3,00 | - | - |
| Propylenglycol | 5,00 | 5,00 | 5,00 | 5,00 |
| Glycerol | 5,00 | 3,00 | 3,00 | 3,00 |
| Methylparaben | 0,20 | 0,20 | 0,20 | 0,20 |
| Tego Carbomer | 0,40 | 0,40 | 0,40 | 0,40 |
| Algenextrakt | 1,00 | - | - | - |
| Caomint | 1,00 | 0,50 | 2,00 | - |
| Calmosensine | 1,00 | 2,00 | - | 2,00 |
| Symdiol 68 | 0,30 | 0,50 | - | - |
| DSH-CN | 5,00 | 5,00 | 5,00 | 5,00 |
| Hydrovance | 4,30 | 2,50 | 8,60 | 10,00 |
| Kombuchka | 3,00 | - | - | 3,00 |
| Glistin | - | 2,00 | - | - |
| Natrulon RC 50 DG | - | - | 1,00 | - |
| Phytokine | 2,00 | 1,50 | 2,00 | 2,00 |
| Ridulisse C | 0,50 | 0,50 | 0,50 | 0,50 |
| Liftiline | 2,00 | 3,00 | 2,00 | 3,00 |
| Matrixyl 3000 | - | 2,00 | - | - |
| Olea europ. Fol extr. S. sicc. | - | - | - | 0,10 |
| Bodyfit | 3,00 | 3,00 | 3,00 | 2,50 |
| Perfume | 0,10 | 0,10 | 0,10 | 0,10 |
| Aqua | ad 100 | ad 100 | ad 100 | ad 100 |

### 2. Hautcremes:

Die Zusammensetzungen 2.1 - 2.3 sind bevorzugte Ausführungsformen von Tagescremes für besonders anspruchsvolle Haut mit einem Anticellulite-Effekt bzw. einem Effekt zur Verbesserung der Silhouette des Gesichts, insbesondere im Kinn-Bereich, mit einem Gehalt an Antiageing-Wirkstoffen.

| | 2.1 | 2.2 | 2.3 |
|---|---|---|---|
| Cetearyl Isononanoate | 4,00 | 4,00 | 4,00 |
| Mineral oil | 6,00 | 6,00 | 6,00 |
| Cutina CBS | 2,00 | 2,00 | 2,00 |
| Palmitic Acid / Stearic Acid | 1,50 | 1,50 | 1,50 |
| Eumulgin B3 | 1,00 | 1,00 | 1,00 |
| Baysilone-Öl M 350 | 1,00 | 1,00 | 1,00 |
| Tocopheryl Acetate | 0,50 | 0,50 | 0,50 |
| Propylparaben | 0,30 | 0,30 | 0,30 |
| Pemulen TR 1 | 0,27 | 0,27 | 0,27 |
| Glycerin | 5,00 | 3,00 | 3,00 |
| Milchsäure 80 % | 0,26 | 0,26 | 0,26 |
| Propylenglycol | 5,00 | 5,00 | 5,00 |
| Methylparaben | 0,30 | 0,30 | 0,30 |
| Phenoxyethanol | 0,90 | 0,90 | 0,90 |
| Phytokine | 2,00 | 1,50 | 2,00 |
| Ridulisse C | 0,50 | 0,50 | 0,50 |
| Liftiline | 2,00 | 3,00 | 2,00 |
| Sepigel 305 | 0,50 | 0,50 | 0,50 |
| Silk Protein | 0,25 | 0,25 | 0,25 |
| Taurin | 1,00 | 0,50 | 2,00 |
| Calmosensine | 1,00 | 2,00 | - |
| Keltrol SF | 0,20 | 0,20 | 0,20 |
| N,N-Bis-(2-hydroxyethyl)harnstoff | 4,30 | 2,50 | 8,60 |
| Perfume | 0,30 | 0,30 | 0,30 |
| Natrium Ascorbylphosphat | 1,00 | - | 1,00 |
| Keratec Pep | - | 2,00 | - |
| Kreatin | - | - | 1,00 |
| Retinol | 0,10 | - | - |
| Deliner | - | 2,00 | - |
| 1,2,9,10-Tetramethoxyaporphin (Glaucin) | 0,01 | - | - |
| Bodyfit | - | 3,00 | 2,50 |
| Wasser | ad 100 | ad 100 | ad 100 |

### 3. Tagescremes:

Die Zusammensetzungen 3.1 - 3.5 sind bevorzugte Ausführungsformen von Tagescremes für besonders anspruchsvolle Haut mit einem Anticellulite-Effekt bzw. einem Effekt zur Verbesserung der Silhouette des Gesichts, insbesondere im Kinn-Bereich, mit einem Gehalt an Antiageing-Wirkstoffen.

| | 3.1 | 3.2 | 3.3 | 3.4 | 3.5 |
|---|---|---|---|---|---|
| Lipoid S 75-3 | 0,50 | 0,50 | 0,50 | 0,50 | 0,50 |
| Isopropylstearate | 4,00 | 4,00 | 4,00 | 4,00 | 4,00 |
| Dibutyl Adipate | 2,00 | 2,00 | 2,00 | 2,00 | 2,00 |
| Tocopheryl Acetate | 0,50 | 0,50 | 0,50 | 0,50 | 0,50 |
| Cutina MD | 1,00 | 1,00 | 1,00 | 1,00 | 1,00 |
| Behenyl Alcohol | 2,00 | 2,00 | 2,00 | 2,00 | 2,00 |
| Baysilone-Öl M 350 | 0,50 | 0,50 | 0,50 | 0,50 | 0,50 |
| Propylparaben | 0,20 | 0,20 | 0,20 | 0,20 | 0,20 |
| Dow Corning 9040 | 1,00 | 1,00 | 1,00 | 1,00 | 1,00 |
| Glycerol | 3,00 | 4,50 | 3,00 | 3,00 | 3,00 |
| Hexandiol | 6,00 | 3,00 | - | - | 4,00 |
| Methylparaben | 0,20 | 0,20 | 0,20 | 0,20 | 0,20 |
| Tego Carbomer | 0,30 | 0,30 | 0,30 | 0,30 | 0,30 |
| Algae Extract | 1,00 | - | - | - | - |
| Photosomes | 0,20 | 0,20 | 0,20 | 0,20 | 0,20 |
| Dimethylmethoxy Chromanol | 0,01 | 0,01 | 0,01 | 0,01 | 0,01 |
| Propylenglycol | 5,00 | 5,00 | 5,00 | 5,00 | 5,00 |
| Phytokine | 2,00 | 1,50 | 2,00 | 2,00 | 1,50 |
| Ridulisse C | 0,50 | 0,50 | 0,50 | 0,50 | 0,50 |
| Liftiline | 2,00 | 3,00 | 2,00 | 2,00 | 3,00 |
| Caomint | 1,00 | - | 2,00 | 2,00 | 2,00 |
| Calmosensine | 1,00 | 2,00 | - | 1,00 | 1,00 |
| Kombuchka | 3,00 | 3,00 | - | - | 2,00 |
| Natrium Ascorbylphosphat | - | - | 1,00 | 1,00 | 1,00 |
| 1,2,9,10-Tetramethoxyaporphin (Glaucin) | 0,005 | 0,01 | 0,0025 | 0,005 | 0,003 |
| Natrulon RC50DG | - | - | - | - | 1,00 |
| Deliner | - | - | 2,00 | 1,00 | - |
| Matrixyl 3000 | 1,00 | 1,00 | - | - | 1,00 |
| Simulgel NS | 1,50 | 1,50 | 1,50 | 1,50 | 1,50 |
| Sodium Benzoate | - | 0,20 | 0,50 | - | - |
| Phenoxyethanol | 0,50 | - | - | - | - |
| Symdiol 68 | 0,30 | 0,50 | 0,30 | - | - |
| TiO₂ | 0,50 | 0,50 | - | - | - |
| Hydrovance | 2,50 | 4,30 | 8,60 | 8,60 | 8,60 |
| Silymarin Phytosome | 0,30 | - | - | - | 0,50 |
| Ectoin | 0,50 | - | 0,50 | 0,50 | 0,50 |
| Vitamin B6 | - | - | 0,01 | 0,01 | 0,01 |
| Perfume | 0,35 | 0,35 | 0,35 | 0,35 | 0,35 |
| Aqua | ad 100 | ad 100 | ad 100 | ad 100 | ad 100 |

Die Zusammensetzungen 3.6 - 3.11 sind bevorzugte Ausführungsformen von pflegenden Cremes mit gelähnlicher Konsistenz mit einem Anticellulite-Effekt bzw. einem Effekt zur Verbesserung der Silhouette des Gesichts, insbesondere im Kinn-Bereich, mit erfindungsgemäß verbesserter Wirkung.

| | 3.6 | 3.7 | 3.8 | 3.9 | 3.10 | 3.11 |
|---|---|---|---|---|---|---|
| 2-Ethylhexylpalmitat | 2,00 | 2,00 | 2,00 | 2,00 | 2,00 | 2,00 |
| Dow Corning 1403 Fluid | 5,00 | 5,00 | 5,00 | 5,00 | 5,00 | 5,00 |
| Synperonic PE L 64 | 2,00 | 2,00 | 2,00 | 2,00 | 2,00 | 2,00 |
| Dow Corning 9040 | 5,00 | 5,00 | 5,00 | 5,00 | 5,00 | 5,00 |
| Dipropylenglykol | 5,00 | 5,00 | 5,00 | 5,00 | 5,00 | 5,00 |
| Tego Carbomer 140 | 0,70 | 0,70 | 0,70 | 0,70 | 0,70 | 0,70 |
| Parfum | 0,35 | 0,30 | 0,35 | 0,35 | 0,30 | 0,30 |
| Phenoxyethanol, rein | 0,40 | 0,40 | 0,40 | 0,40 | 0,40 | 0,40 |
| Natrulon RC-50DG | - | 0,75 | 0,75 | 0,75 | 0,75 | 0,75 |
| Menthyl Lactate | 0,50 | 0,50 | 0,50 | 0,50 | 0,50 | 0,50 |
| Ethanol 96 % DEP vergällt | 5,00 | 5,00 | 5,00 | 5,00 | 5,00 | 5,00 |
| Coffein wasserfrei | 0,50 | - | 0,50 | 0,50 | 0,50 | 0,50 |
| Ridulisse C | - | - | - | 0,50 | - | - |
| Bodyfit | 2,50 | 2,50 | 2,50 | 2,50 | 2,50 | 2,50 |
| Simulgel NS | 0,75 | 0,75 | 0,75 | 0,75 | 0,75 | 0,75 |
| Matrixyl | - | - | - | - | 2,00 | - |
| Matrixyl 3000 | - | - | - | - | - | 2,00 |
| Natriumhydroxid Perlen | 0,081 | 0,081 | 0,081 | 0,081 | 0,081 | 0,081 |
| Wasser, vollentsalzt | ad 100,00 | ad 100,00 | ad 100,00 | ad 100,00 | ad 100,00 | ad 100,00 |

### 4. Beispielserie:

Die Zusammensetzungen 4.1 - 4.5 sind bevorzugte Ausführungsformen von Tagescremes mit höherem Lichtschutzfilter (SPF ca. 10 - 15) für besonders anspruchsvolle Haut mit einem Anticellulite-Effekt bzw. einem Effekt zur Verbesserung der Silhouette des Gesichts, insbesondere im Kinn-Bereich, mit einem Gehalt an Antiageing-Wirkstoffen.

| | 4.1 | 4.2 | 4.3 | 4.4 | 4.5 |
|---|---|---|---|---|---|
| Montanov 68 | 5,00 | 5,00 | 5,00 | 5,00 | 5,00 |
| Myritol 318 | 5,00 | 5,00 | 5,00 | 5,00 | 5,00 |
| Cetiol SB 45 | 0,50 | 0,50 | 0,50 | 0,50 | 0,50 |
| Novata AB | 2,00 | 2,00 | 2,00 | 2,00 | 2,00 |
| Stenol 1618 | 1,00 | 1,00 | 1,00 | 1,00 | 1,00 |
| Baysilon M 350 | 0,50 | 0,50 | 0,50 | 0,50 | 0,50 |
| Tocopherylacetat | 0,50 | 0,50 | 0,50 | 0,50 | 0,50 |
| Controx KS | 0,25 | 0,25 | 0,25 | 0,25 | 0,25 |
| Parsol SLX | - | 4,00 | 4,00 | - | - |
| Parsol HS | - | 2,00 | 2,00 | 2,00 | - |
| Neo Heliopan AP | - | - | - | 1,00 | - |
| Parsol 340 | - | - | - | 5,00 | - |
| Uvinul MBC 95 | 2,00 | - | - | - | - |
| Parsol1789 | 1,00 | 1,80 | 1,80 | - | - |
| Propylparaben | 0,20 | 0,20 | 0,20 | 0,20 | 0,20 |
| Tego Carbomer 140 | 0,50 | 0,50 | 0,50 | 0,50 | 0,50 |
| 1,6-Hexandiol | 6,00 | 6,00 | 3,00 | 6,00 | - |
| Talkum | 0,50 | 0,50 | 0,50 | 0,50 | 0,50 |
| Methylparaben | 0,20 | 0,20 | 0,20 | 0,20 | 0,20 |
| Glycerin | 4,50 | 4,50 | 3,00 | 4,50 | 3,00 |
| Dry Flo Plus | 1,00 | 1,00 | 1,00 | 1,00 | 1,00 |
| Natipide 2 PG | 1,00 | 1,00 | 1,00 | 1,00 | 1,00 |
| Phenoxyethanol | 0,40 | 0,40 | 0,40 | 0,40 | 0,40 |
| Bodyfit | 2,00 | 2,50 | 4,00 | 3,00 | 3,00 |
| Coffein | 0,50 | 1,00 | - | - | 0,35 |
| Carnitin | - | 0,10 | 0,50 | - | - |
| Matrixyl 3000 | 1,00 | - | - | 4,00 | 2,00 |
| Ridulisse C | - | - | 3,00 | - | 0,50 |
| Parfum | 0,40 | 0,40 | 0,40 | 0,40 | 0,40 |
| Aqua | ad 100 | ad 100 | ad 100 | ad 100 | ad 100 |

### 5. Wasser-in-Öl-Emulsion

Die Zusammensetzungen 5.1 - 5.3 sind bevorzugte Ausführungsformen von Cremes mit einem Anticellulite-Effekt bzw. einem Effekt zur Verbesserung der Silhouette des Gesichts, insbesondere im Kinn-Bereich, auf der Basis einer reichhaltigen Wasser-in-Öl-Emulsion, die in die Haut einmassiert werden kann und dadurch einen zusätzlichen Bonuseffekt hervorruft.

| | 5.1 | 5.2 | 5.3 |
|---|---|---|---|
| Lameform TGI | 3,00 | 3,00 | 3,00 |
| Elfacos ST 37 | 0,50 | 0,50 | 0,50 |
| Microcrystalline Wax | 3,00 | 3,00 | 3,00 |
| Softisan 649 | 1,00 | 1,00 | 1,00 |
| Paraffinöl | 8,00 | 8,00 | 8,00 |
| Vaseline | 2,00 | 2,00 | 2,00 |
| Vitamin E Acetat | 2,00 | 2,00 | 2,00 |
| Methylparaben | 0,30 | 0,30 | 0,30 |
| Propylparaben | 0,30 | 0,30 | 0,30 |
| Isopropylisostearat | 8,00 | 8,00 | 8,00 |
| 1,3-Butylenglycol | 5,00 | 6,00 | 7,00 |
| Bodyfit | 2,50 | 4,00 | 3,00 |
| Coffein | 0,50 | 0,50 | - |
| Carnitin | - | 0,10 | 0,50 |
| Milchsäure 80%ig | 0,60 | 0,60 | 0,60 |
| Panthenol | 0,5 | 1,0 | 0,5 |
| Tephrosia pupurea Seed Extract | 0,1 | - | - |
| Hydrovance | 2,00 | 4,00 | - |
| Parfum | 0,2 | 0,2 | 0,2 |
| Water | ad 100 | ad 100 | ad 100 |

### 6. Gesichtswasser

Die Zusammensetzungen 6.1 - 6.3 sind bevorzugte Ausführungsformen von Gesichtswässern mit einem straffenden Effekt zur Verbesserung der Silhouette des Gesichts und der Ausstrahlung des Teints, insbesondere bei müder Haut.

| | 6.1 | 6.2 | 6.3 |
|---|---|---|---|
| Dipropylenglycol | 10,00 | 10,00 | 10,00 |
| Chlorhexidindigluconat | 1,00 | 1,00 | 1,00 |
| Synperonic PE7 L 64 | 3,00 | 3,00 | 3,00 |
| D-Panthenol | 0,50 | 0,50 | 0,50 |
| Hydagen CMF | 3,00 | 3,00 | 3,00 |
| PEG-40 Hydrogenated Castor Oil / Trideceth 9 / Propylene Glycol | 0,50 | 0,50 | 0,50 |
| Caomint | 0,50 | 0,50 | 0,50 |
| Bodyfit | 1,00 | 0,25 | 0,50 |
| Coffein | 0,20 | - | - |
| Ridulisse C | 1,00 | - | - |
| Fucogel 1000 | - | - | 1,00 |
| Betain | - | 1,00 | - |
| Parfum | 0,20 | 0,20 | 0,20 |
| Aqua | ad 100 | ad 100 | ad 100 |

### 7. Wasser-in-Silicon-Emulsionen

Die Zusammensetzungen 7.1 - 7.3 sind bevorzugte Ausführungsformen von Cremes mit einem Anticellulite-Effekt bzw. einem Effekt zur Verbesserung der Silhouette des Gesichts, insbesondere im Kinn-Bereich, mit erfindungsgemäß verbesserter Hautverträglichkeit, auf der Basis einer reichhaltigen und dennoch nicht-fettenden Wasser-in-Silicon-Emulsion, die in die Haut einmassiert werden kann.

| | 7.1 | 7.2 | 7.3 |
|---|---|---|---|
| Belsil DM 100 | 2,50 | 2,50 | 2,50 |
| Dow Corning 245 Fluid | 25,00 | 25,00 | 25,00 |
| Abil EM 90 | 2,00 | 2,00 | 2,00 |
| Natriumchlorid | 2,00 | 2,00 | 2,00 |
| Symdiol 68 | 0,30 | 0,30 | 0,30 |
| Phenoxyethanol | 0,40 | 0,40 | 0,40 |
| Bodyfit | 3,00 | 4,00 | 2,00 |
| Coffein | - | 0,20 | - |
| Ridulisse C | - | 2,00 | 1,00 |
| Rhamnosoft | 2,00 | - | - |
| Hydrovance | - | 3,00 | - |
| Folsäure | - | - | 0,10 |
| Parfum | 0,30 | 0,30 | 0,30 |
| Aqua | ad 100 | ad 100 | ad 100 |

### 8. Beispielserie:

Die Zusammensetzungen 8.1 - 8.4 sind bevorzugte Ausführungsformen von Tagescremes für besonders anspruchsvolle Haut mit einem Anticellulite-Effekt bzw. einem Effekt zur Verbesserung der Silhouette des Gesichts, insbesondere im Kinn-Bereich, mit einem erhöhten Gehalt an diversen Antiageing-Wirkstoffen.

| | 8.1 | 8.2 | 8.3 | 8.4 |
|---|---|---|---|---|
| Distelöl | 3,00 | 3,00 | 3,00 | 3,00 |
| Myritol 318 | 5,00 | 5,00 | 5,00 | 5,00 |
| Novata AB | 2,00 | 2,00 | 2,00 | 2,00 |
| Behenyl Alcohol | 1,00 | 1,00 | 1,00 | 1,00 |
| Cutina MD | 2,00 | 2,00 | 2,00 | 2,00 |
| Cetearyl Alcohol | 1,00 | 1,00 | 1,00 | 1,00 |
| Isopropylstearat | 4,00 | 4,00 | 4,00 | 4,00 |
| Shea Butter | 2,00 | 2,00 | 2,00 | 2,00 |
| Baysilone-Öl M 350 | 1,00 | 1,00 | 1,00 | 1,00 |
| Controx KS | 0,05 | 0,05 | 0,05 | 0,05 |
| Propylparaben | 0,20 | 0,20 | 0,20 | 0,20 |
| Dow Corning 1501 Fluid | 1,00 | 1,00 | 1,00 | 1,00 |
| Dry Flo Plus | 1,00 | 1,00 | 1,00 | 1,00 |
| TiO₂ | 0,50 | 0,50 | 0,50 | 0,50 |
| Hexandiol | 6,00 | 3,00 | - | - |
| Propylenglycol | 5,00 | 5,00 | 5,00 | 5,00 |
| Glycerol | 5,00 | 3,00 | 3,00 | 3,00 |
| Methylparaben | 0,20 | 0,20 | 0,20 | 0,20 |
| Tego Carbomer | 0,40 | 0,40 | 0,40 | 0,40 |
| Algenextrakt | 1,00 | - | - | - |
| Symdiol68 | 0,30 | 0,50 | - | - |
| DSH-CN | 5,00 | 5,00 | 5,00 | 5,00 |
| Hydrovance | 4,30 | 2,50 | 8,60 | 10,00 |
| Bodyfit | 3,00 | 4,00 | 2,80 | 3,00 |
| Coffein | - | 0,30 | - | 0,10 |
| Natrulon RC 50 DG | - | - | 1,00 | - |
| Phytokine | 2,00 | | - | - |
| Matrixyl 3000 | - | 2,00 | - | - |
| Ridulisse C | - | - | 2,00 | 2,50 |
| Argireline | 1,00 | - | - | - |
| Sepilift DPHP | - | 0,10 | - | - |
| Modulene | - | - | 1,00 | - |
| Collaxyl | - | - | - | 2,00 |
| Ederline H | 2,00 | - | - | - |
| Perfume | 0,10 | 0,10 | 0,10 | 0,10 |
| Aqua | ad 100 | ad 100 | ad 100 | ad 100 |

### 9. Hautcremes:

Die Zusammensetzungen 9.1 - 9.3 sind weitere bevorzugte Ausführungsformen von Cremes für besonders anspruchsvolle Haut mit einem Anticellulite-Effekt bzw. einem Effekt zur Verbesserung der Silhouette des Gesichts, insbesondere im Kinn-Bereich, mit einem erhöhten Gehalt an diversen Antiageing-Wirkstoffen.

| | 9.1 | 9.2 | 9.3 |
|---|---|---|---|
| Cetearyl Isononanoate | 4,00 | 4,00 | 4,00 |
| Mineral oil | 6,00 | 6,00 | 6,00 |
| Cutina CBS | 2,00 | 2,00 | 2,00 |
| Cutina FS 45 | 1,50 | 1,50 | 1,50 |
| Eumulgin B3 | 1,00 | 1,00 | 1,00 |
| Baysilone-Öl M 350 | 1,00 | 1,00 | 1,00 |
| Tocopherylacetat | 0,50 | 0,50 | 0,50 |
| Propylparaben | 0,30 | 0,30 | 0,30 |
| Pemulen TR 1 | 0,27 | 0,27 | 0,27 |
| 1,3-Butylenglycol | 5,00 | 3,00 | 3,00 |
| Milchsäure 80 % | 0,26 | 0,26 | 0,26 |
| Propylenglycol | 5,00 | 5,00 | 5,00 |
| Methylparaben | 0,30 | 0,30 | 0,30 |
| Phenoxyethanol | 0,90 | 0,90 | 0,90 |
| Pantolacton | 0,50 | - | - |
| Seanergilium BG | - | 1,00 | - |
| Natriumchlorid | 0,05 | 0,05 | 0,05 |
| Sepigel 305 | 0,50 | 0,50 | 0,50 |
| Calmosensine | 1,00 | 2,00 | - |
| Keltrol SF | 0,20 | 0,20 | 0,20 |
| Hydrovance | 4,30 | 2,50 | 8,60 |
| Natriumascorbylphosphat | 1,00 | - | - |
| Keratec Pep | - | 2,00 | - |
| Kreatin | - | - | 0,10 |
| Retinol | 0,10 | - | - |
| Bodyfit | 3,00 | 3,00 | 2,00 |
| Coffein | 0,20 | - | - |
| Perfume | 0,30 | 0,30 | 0,30 |
| Aqua | ad 100 | ad 100 | ad 100 |

### 10. Hautcremes auf Basis einer Lipoprotein-Creme

Die Zusammensetzungen 10.1 - 10.6 sind bevorzugte Ausführungsformen von Cremes mit einem Anticellulite-Effekt bzw. einem Effekt zur Verbesserung der Silhouette des Gesichts, insbesondere im Kinn-Bereich, mit besonderer Hautverträglichkeit und einem Gehalt an diversen Antiageing-Wirkstoffen, auf Basis einer besonders milden Emulsionsbasis mit einem natürlichen Protein-Emulgator (Hibiscin^{®} HP LS 9198).

| | 10.1 | 10.2 | 10.3 | 10.4 | 10.5 | 10.6 |
|---|---|---|---|---|---|---|
| Myritol^{®} PC | 3,5 | 3,5 | 3,5 | 3,5 | 3,5 | 3,5 |
| Lanette^{®} 22 | 3,0 | 3,0 | 3,0 | 3,0 | 3,0 | 3,0 |
| Cutina^{®} GMS-V | 3,0 | 3,0 | 3,0 | 3,0 | 3,0 | 3,0 |
| Stenol^{®} 16/18 | 2,0 | 2,0 | 2,0 | 2,0 | 2,0 | 2,0 |
| Distelöl | 3,0 | 3,0 | 3,0 | 3,0 | 3,0 | 3,0 |
| Controx^{®} KS | 0,05 | 0,05 | 0,05 | 0,05 | 0,05 | 0,05 |
| Baysilon^{®} M350 | 1,0 | 1,0 | 1,0 | 1,0 | 1,0 | 1,0 |
| Isopropylstearat | 6,0 | 6,0 | 6,0 | 6,0 | 6,0 | 6,0 |
| Propylparaben | 0,2 | 0,2 | 0,2 | 0,2 | 0,2 | 0,2 |
| 1,3-Butylenglycol | 5,0 | 5,0 | 5,0 | 5,0 | 5,0 | 5,0 |
| 1,6-Hexandiol | 5,0 | 5,0 | 5,0 | 5,0 | 5,0 | 5,0 |
| Methylparaben | 0,2 | 0,2 | 0,2 | 0,2 | 0,2 | 0,2 |
| Hibiscin^{®} HP LS 9198 | 3,0 | 3,0 | 3,0 | 3,0 | 3,0 | 3,0 |
| Citronensäure | 0,1 | 0,1 | 0,1 | 0,1 | 0,1 | 0,1 |
| Simulgel^{®} NS | 2,0 | 2,0 | 2,0 | 2,0 | 2,0 | 2,0 |
| Pearl Protein Extract | - | - | 2,00 | - | - | 1,00 |
| Bodyfit | 3,0 | 3,0 | 3,0 | 4,0 | 3,0 | 3,0 |
| Coffein | 0,30 | 0,30 | 0,30 | - | - | - |
| Ridulisse C | 1,00 | 0,50 | 1,00 | - | - | - |
| Carnitin | 0,20 | - | - | 0,50 | 0,30 | 0,30 |
| Matrixyl 3000 | 3,00 | - | - | - | 2,00 | |
| Aqua | ad 100 | ad 100 | ad 100 | ad 100 | ad 100 | ad 100 |

### 11. Reinigungsgele mit Slimming-Effekt bzw. Anticellulite-Effekt:

Die Zusammensetzungen 11.1 - 11.3 sind bevorzugte Ausführungsformen von Reinigungsgelen mit einem Anticellulite-Effekt bzw. einem Effekt zur Verbesserung der Silhouette des Gesichts, insbesondere im Kinn-Bereich, mit erfindungsgemäß verbesserter Hautverträglichkeit.

| | 11.1 | 11.2 | 11.3 |
|---|---|---|---|
| Carbomer | 1,40 | 1,40 | 1,40 |
| 1,3-Butylenglycol | 4,00 | 4,00 | 4,00 |
| Sodium Benzoate | 0,40 | 0,40 | 0,40 |
| Plantaren^{®} 1200 | 7,50 | 7,50 | 7,50 |
| Dehyton^{®} K | 3,40 | 3,40 | 3,40 |
| Texapon^{®} SB 3 | 5,00 | 5,00 | 5,00 |
| Cetiol^{®} HE | 0,50 | 0,50 | 0,50 |
| Lamesoft^{®} PO 65 | 5,00 | 5,00 | 5,00 |
| Controx KS | 0,05 | 0,05 | 0,05 |
| Sodium pyrrolidone carboxylic acid | 1,60 | 1,60 | - |
| Pantolactone | 1,00 | 1,00 | - |
| Tetrasodium EDTA | 0,25 | 0,25 | 0,25 |
| Sodium Lactate | 1,80 | - | - |
| Panthenol | 0,50 | 0,50 | 0,50 |
| Bodyfit | 3,0 | 3,0 | 3,0 |
| Coffein | 0,30 | 0,30 | 0,30 |
| Ridulisse C | 1,00 | - | - |
| Carnitin | 0,20 | - | 0,20 |
| Perfume | 0,40 | 0,40 | 0,40 |
| Aqua | ad 100 | ad 100 | ad 100 |

### 12. Zweiphasen-Anticellulite-Massagefluid für die Körperhaut

Die Zusammensetzungen 12.1 und 12.2 sind bevorzugte Ausführungsformen von Massagefluiden mit einem Anticellulite-Effekt bzw. einem Effekt zur Verbesserung der Silhouette des Gesichts, insbesondere im Kinn-Bereich, mit erfindungsgemäß verbesserter Hautverträglichkeit, auf Basis einer Zweiphasenzusammensetzung, die direkt vor dem Gebrauch durch Schütteln in eine Emulsion überführt wird und sich besonders leicht in die Haut einmassieren lässt. Die Zusammensetzung ruft ein angenehm erfrischendes Hautgefühl hervor.

| | 12.1 | 12.2 |
|---|---|---|
| Ocaline | 1,000000 | 1,000000 |
| Milchsäure 80% DAB | 0,100000 | 0,100000 |
| Dinatriumphosphat wasserfrei | 0,100000 | 0,100000 |
| D-Panthenol 75 % | 0,100000 | 0,100000 |
| Parfum | 0,150000 | 0,150000 |
| Brij 30 | 0,500000 | 0,500000 |
| Trisiloxane Fluid 1 cs | 20,000000 | 20,000000 |
| Bodyfit | 3,000000 | 2,500000 |
| Coffein | 0,500000 | - |
| Theobromin | | 0,300000 |
| Ridulisse C | 1,000000 | - |
| Carnitin | 0,200000 | - |
| Ethanol 96 % | 10,000000 | 10,000000 |
| Wasser, vollentsalzt | ad 100,000000 | ad 100,000000 |

### 13. Beispiele für Zusammensetzungen zum Tränken von Tüchern

| | Bestandteile | 13.1 | 13.2 | 13.3 | 13.4 |
|---|---|---|---|---|---|
| Phase 1 | PEG-40 Hydrogenated Castor Oil | 0,1 | 0,1 | 0,1 | - |
| | Trideceth-9 | 0,1 | 0,1 | 0,1 | - |
| | Parfum | 0,3 | 0,1 | 0,1 | 0,1 |
| Phase 2 | Hexyllaurat | 2,0 | - | - | - |
| | Dicaprylylcarbonat | - | - | 2,0 | - |
| | Dipropylenglycol | - | 2,0 | - | - |
| Phase 3 | Allantoin | 0,5 | - | - | - |
| | Citronensäure (Monohydrat) | 1,5 | 1,5 | 1,5 | 1,5 |
| | Trinatriumcitrat (Dihydrat) | 2,0 | 2,0 | 2,0 | 2,0 |
| | Decylglucosid | - | - | - | 2,0 |
| | Pemulen TR 1 | - | - | - | 0,2 |
| | Bodyfit | 3,0 | 3,0 | 3,0 | 4,0 |
| | Coffein | 0,30 | 0,30 | 0,30 | - |
| | Ridulisse C | 1,00 | 0,50 | 1,00 | - |
| | Carnitin | 0,20 | - | - | 0,50 |
| | Wasser | ad 100 | ad 100 | ad 100 | ad 100 |

Mit diesen Tränkzusammensetzungen wurden verschiedene Viskose-Vliesstoffe ausgerüstet. Es wurden gelochte, ungelochte, genoppte, einlagige, zweilagige und dreilagige Vliese ausgerüstet. Die Tücher wurden sowohl als feuchte Tücher als auch als trockene Tücher (das heißt, nach dem Ausrüsten wurden die Tücher bis auf einen Restwassergehalt kleiner 10 Gew.-%, bevorzugt kleiner 5 Gew.-%, getrocknet, um direkt vor Gebrauch mit einer kleinen Menge an Wasser wieder befeuchtet zu werden oder auf feuchter Haut angewendet zu werden) verwendet.
Als besonders bevorzugt erwiesen sich genoppte Tücher (beispielsweise mit heiß aufgesprühten Kunststoffnoppen) und Tücher mit grober Oberflächenstruktur, da diese den gewünschten Massageeffekt besonders vorteilhaft unterstützen.
Derartige Slimming-Tücher sind besonders vorteilhaft zur Anwendung unterwegs geeignet.

### 14. Matrixpflaster mit Anti-Cellulite-Effekt

| | 14.1 | 14.2 | 14.3 | 14.4 | 14.5 |
|---|---|---|---|---|---|
| DURO-TAK^{®} 387-2516 | 76 | 76 | 76 | 76 | 76 |
| Aloe Vera Gel | 1 | - | 1 | - | - |
| Propylenglycolmonooleat | 5 | 5 | - | - | - |
| Tween^{®}-80 | - | - | 5 | 3 | 5 |
| Natriumcitrat | 0,1 | 0,1 | 0,1 | 0,1 | 0,1 |
| Controx^{®} KS | 0,05 | 0,05 | 0,05 | 0,05 | 0,05 |
| Bodyfit | 3,0 | 3,0 | 3,0 | 4,0 | 3,0 |
| Coffein | 0,30 | 0,30 | 0,30 | - | - |
| Ridulisse C | 1,00 | 0,50 | 1,00 | - | - |
| Carnitin | 0,20 | - | - | 0,50 | 0,30 |
| Matrixy13000 | 3,00 | - | - | - | 2,00 |
| Wasser | ad 100 | ad 100 | ad 100 | ad 100 | ad 100 |

### 15. Reservoirpflaster mit Anti-Cellulite-Effekt

| Bestandteile des Wirkstoffreservoirs | 15.1 | 15.2 |
|---|---|---|
| Bodyfit | 3,0 | 3,0 |
| Coffein | 0,30 | 0,30 |
| Ridulisse C | - | 0,50 |
| Carnitin | 0,50 | - |
| Ederline H | 1,0 | 1,0 |
| Carbopol^{®} 980 | 0,5 | - |
| Pemulen^{®}TR1 | - | 0,5 |
| NaOH | 0,1 | 0,1 |
| Titandioxid | 0,2 | 0,2 |
| Ethanol | 1,0 | 1,0 |
| Polyvinylalkohol | 2,0 | 1,0 |
| Carboxymethylcellulose | 1,0 | 0,5 |
| Glycerin | 10 | 20 |
| Sorbitol | 7,0 | 5,0 |
| 1,3-Butandiol | 3,0 | 3,0 |
| Tween^{®}-80 | 0,05 | 0,05 |
| Paraffinöl | 5,0 | 2,0 |
| Natriumcitrat | 0,1 | 0,1 |
| Controx KS | 0,05 | 0,05 |
| Wasser | ad 100 | ad 100 |

Die Bestandteile der Wirkstoffreservoir-Rezepturen 15.1 bzw. 15.2 wurden miteinander vermischt, so dass eine gelartige Masse entstand. Mit dieser Masse wurden Pflasterträger beschichtet, so dass sogenannte Reservoirpflaster erhalten wurden. Diese Pflaster werden sowohl auf die trockene Haut als auch auf die angefeuchtete Haut gelegt und verbleiben dort für eine Zeit von wenigen Sekunden bis einigen Stunden.

### 16. Körpercremes

Die nachfolgenden Rezepturen 16.1 und 16.2 sind Beispiele für erfindungsgemäße Anticellulite-Körpercremes. Diese werden bevorzugt in die Haut der Oberschenkel und Gesäßpartien einmassiert.

| | 16.1 | 16.2 |
|---|---|---|
| Paraffinum Liquidum | 20,00 | 20,00 |
| Arlacel 165 | 4,00 | 4,00 |
| Eutanol G | 1,00 | 1,00 |
| Cetiol SN | 1,50 | 1,50 |
| Cremophor A 6 | 2,00 | 2,00 |
| Behenylalkohol | 1,60 | 1,60 |
| Silikonöl 350 cs | 3,00 | 3,00 |
| Brij 721 VP | 0,40 | 0,40 |
| Propylparaben | 0,20 | 0,20 |
| Controx KS | 0,25 | 0,25 |
| Propandiol-1,2 | 3,00 | 3,00 |
| Glycerin 86% pflanzlich | 5,00 | 5,00 |
| Polyethylenglykol MG 400 | 1,70 | 1,70 |
| Methylparaben | 0,20 | 0,20 |
| Phenoxyethanol, rein | 0,50 | 0,50 |
| Carbopol ETD 2020 | 0,50 | 0,50 |
| Natriumhydroxid Perlen | 0,03 | 0,03 |
| Citronensäure Monohydrat | 0,10 | 0,10 |
| Trinatriumcitrat Dihydrat | 0,10 | 0,10 |
| Bodyfit | 3,00 | 3,00 |
| Coffein | 0,50 | 0,50 |
| Carnitin | 0,20 | 0,20 |
| Herbasol Extrakt Capsicum | - | 0,50 |
| Parfum | 0,30 | 0,20 |
| Simulgel NS | 1,00 | 1,10 |
| Wasser | ad 100,00 | ad 100,00 |

### Liste der verwendeten Rohstoffe

| Handelsname | INCI-Bezeichnung | Lieferant/Hersteller |
|---|---|---|
| | | |
| Abil EM 90 | Cetyl PEG/PPG-10/1 Dimethicone | Degussa |
| Argireline | Acetyl-Hexapeptide-3 | Lipotec |
| Arlacel 165 | Glyceryl Stearate, PEG-100 Stearate | Uniqema |
| Baysilone-Öl M 350 | Dimethicone (350 cSt) | GE Bayer Silicones |
| Belsil DM 100 | Dimethicone (100 cSt) | Wacker |
| Bodyfit | Glycerin, Aqua (Water), Cocoglucoside, Caprylyl Glycol, Alcohol, Glaucine (0,1 - 1 Gew.-% Glaucin) | Sederma |
| Brij 30 | Laureth-4 | Uniqema |
| Brij 721 VP | STEARETH-21 | Uniqema |
| Calmiskin | Water (and) Mentha piperita (Peppermint) Leaf Extract | Silab |
| Calmosensine | BUTYLENE GLYCOL, WATER, LAURETH-3, HYDROXYETHYLCELLULOSE, Acetyl Dipeptide-1 Cetylester | Sederma |
| Caomint | Propylene Glycol, Aqua, Mentha piperita, Theobroma cacao | Solabia |
| Carbopol 980 | Carbomer | Noveon |
| Carbopol ETD 2020 | Acrylates/C10-30 Alkyl Acrylate Crosspolymer | Noveon |
| Cetiol^{®} B | Dibutyl Adipate | Cognis |
| Cetiol^{®} HE | PEG-7 Glyceryl Cocoate | Cognis |
| Cetiol^{®} SB 45 | Butyrospermum parkii (Shea butter) | Cognis |
| Cetiol^{®} SN | Cetearyl Isononanoate | Cognis |
| Controx KS | Tocopherol, Hydrogenated Palm Glycerides Citrate | Cognis |
| Cremophor A 6 | CETEARETH-6, STEARYL ALCOHOL | BASF |
| Cutina CBS | Glyceryl Stearate / Cetearyl Alcohol / Cetyl Palmitate / Cocoglycerides im Gewichtsverhältnis 7:1:1:1 | Cognis |
| Cutina GMS | Glyceryl Stearate (Mischung aus Glycerylmono- und distearat) | Cognis |
| Cutina MD | Glyceryl Stearate (Mischung aus Glycerylmono- und distearat) | Cognis |
| Deliner | Zea Mays (Corn) Kernel Extract, Butylene Glycol, Xanthan Gum | Coletica |
| Dow Corning 1501 Fluid | Cyclomethicone (85 - 86 Gew.-%), Dimethiconol (14-15 Gew.-%) | Dow Corning |
| Dow Corning 200 | Dimethicone (0,65 cSt) | Dow Corning |
| Dow Corning 9040 | Cyclomethicone/ Dimethicone Crosspolymer (87-88%/12-13 %) | Dow Corning |
| Dow Corning^{®}200 Fluid | Dimethicone (5 cSt) | Dow Corning |
| Dow Corning^{®}245 Fluid | Cyclomethicone | Dow Corning |
| Dry Flo Plus | Aluminium Starch Octenylsuccinate | National Starch |
| DSH CN | Water, Dimethylsilanol Hyaluronate | Exsymol |
| DURO-TAK^{®} 387-2516 | Polyacrylate, Ethylcellulose, Polyvinylpyrrolidon | National Starch and Chemical |
| EDENOR L2 S M | Palmitic Acid, Stearic Acid (ca. 1: 1) | Cognis |
| Ederline H | PEG-40 Hydrogenated Castor Oil, PPG-2-Ceteareth-9, Pyrus Malus (Apple) Fruit Extract | Seporga |
| Elfacos ST 37 | PEG-45/Dodecyl Glycol Copolymer | Akzo Nobel |
| EMULGADE^{®} SE | Glyceryl Stearate, Ceteareth-20, Cetea-reth-12, Cetearyl Alcohol, Cetyl Palmitate | Cognis |
| Enteline 2 | Butylene Glycol, Glycerine, Hydrolyzed Enteromorpha compressa | BiotechMarine |
| Eumulgin B 3 | Ceteareth-30 | Cognis |
| Eusolex^{®} OCR | Octocrylene | Merck KGaA |
| Eutanol G | OCTYLDODECANOL | Cognis |
| Exsy Algine | ACETYL CITRULL AMIDO ARGININE | Exsymol |
| Fucogel 1000 | Biosaccharide Gum-1 | Solabia |
| Glistin | Water, L-Glutamylaminoethyl indole | Exsymol |
| Herbasol Extrakt Capsicum | Propylene Glycol, Aqua (Water), Capsicum Frutescens Fruit Extract, Sorbitol | Cosmetochem |
| Hibiscin^{®} HP-LS-9198 | Water, Hibiscus esculentus Seed Extract, Phenoxyethanol | Laboratoires Sérobiologiques |
| Hydagen CMF | Chitosan Glycolate | Cognis |
| Hydrovance | Wasser, Bis-N,N'-(2-Hydroxyethyl)harnstoff, Harnstoff, Ammoniumlactat | National Starch |
| Keltrol SF | Xanthan Gum | Kelco |
| Keratec Pep | Water, Hydrolyzed Keratin | Croda |
| Keratolite | AQUA (WATER), MALIC ACID, BUTYLENE GLYCOL, PRUNUS AMYGDALUS DULCIS (SWEET ALMOND) FRUIT EXTRACT, SODIUM BENZOATE, PHENOXYETHANOL, METHYLPARABEN, PROPYLPARABEN, ETHYLPARABEN | Coletica |
| Kombuchka | Saccharomyces/Xylinum/Black Tea Ferment, Glycerin, Hydroxyethylcellulose | Sederma |
| Lameform TGI | Polyglyceryl-3 Diisostearate | Cognis |
| Lamesoft^{®} PO 65 | Coco-Glucoside, Glyceryl Oleate, Water | Cognis |
| LANETTE^{®} 22 | Behenyl Alcohol | Cognis |
| LANETTE^{®} O | Cetearyl Alcohol | Cognis |
| Liftiline | Aqua, Hydrolyzed Wheat Protein (7 - 10 Gew.-% Aktivsubstanz) | Silab |
| Lipochroman 6 | DIMETHYLMETHOXY CHROMANOL | Lipotec |
| Lipoid S 75-3 | Aqua, Lecithine | Lipoid GmbH |
| Matrixyl | Aqua, Palmitoyl Pentapeptide-3 | Sederma |
| Matrixyl 3000 | Glycerin, Aqua, Butylene Glycol, Carbomer, Polysorbate 20, Palmitoyl Oligopeptide, Palmitoyl Tetrapeptide-1 | Sederma |
| Montanov 68 | Cetearyl Alkohol, Cetearyl Glucoside | Seppic |
| Montanov L | C14-22 Alcohols/C12-20 Alkyl Glucoside | Seppic |
| Myritol 318 | Caprylic/Capric Triglyceride | Cognis |
| MYRITOL^{®}PC | PROPYLENE GLYCOL DICAPRYLATE/DICAPRATE | Cognis |
| Natipide II PG | Aqua, Propylene Glycol, Lecithin | Phospholipid GmbH |
| Natrulon RC 50 DG | WATER, CARNITINE, POLYGLYCERIN-10 | Lonza |
| Neo Heliopan AP | Disodium Phenyl Dibenzimidazole Tetrasulfonate | Symrise |
| Novata AB | Cocoglycerides | Cognis |
| Ocaline | Sea Water (and) Water (and) Cucurbita pepo (pumpkin) seed extract | Soliance |
| Olea europ. Fol extr. S. sicc. | Olea Europea (Olive) Leaf Extract | Fruitarom |
| Omega-CH-Aktivator | Tripeptide-1 | GfN |
| Parsol 1789 | BUTYL METHOXYDIBENZOYLMETHANE | DSM |
| Parsol 340 | Octocrylene | DSM |
| Parsol HS | PHENYLBENZIMIDAZOLE SULFONIC ACID | DSM |
| Parsol SLX | Polysilicone-15 | DSM |
| Pearl Protein Extract | Aqua, Hydrolyzed Conchiolin Protein | Maruzen |
| Pemulen TR 1 | Acrylates/C10-30 Alkyl Acrylate Crosspolymer | Noveon |
| Photosomes | Aqua, Lecithine, Plancton Extract | AGI Dermatics |
| Phytokine | Aqua, Butylene Glycol, Hydrolyzed Soy Protein, Methylparaben, Ethylparaben, Propylparaben (0,4 - 0,8 Gew.-% Aktivsubstanz) | Coletica |
| Plantaren^{®} 1200 | Lauryl Glucoside, ca. 50 % Aktivsubstanz | Cognis |
| Rhamnosoft | Biosaccharide Gum-2 | Solabia |
| Ridulisse C | Water, HYDROLYZED SOY PROTEIN (3 - 5 Gew.-% Aktivsubstanz) | Silab |
| Seanergilium BG | Butylene Glicol, Laminaria Digitata Extract, Methylparaben | Coletica |
| Sepigel^{®}305 | Aqua (Water) Polyacrylamide, C₁₃-C₁₄ Isoparaffin, Laureth-7 (Aktivsubstanz Verdickerpolymer ca. 45 - 49 Gew.-%) | SEPPIC |
| Sepilift DPHP | Dipalmitoyl Hydroxyproline | Seppic |
| Silymarin Phytosome | Silybum Marianum Extract and Phospholipids | Indena SpA |
| Simulgel NS | Aqua (Water)/Hydroxyethyl Acrylate / Sodium Acryloyldimethyl Taurate Copolymer / Squalane / Polysorbate 60 | Seppic |
| Softisan 649 | Bis-Diglyceryl Polyacyladipate-2 | Sasol |
| Stenol 16/18 | Cetearylalkohol | Cognis |
| Symdiol 68 | 1,2-Octandiol, 1,2-Hexandiol | Symrise |
| Synperonic PE L 64 | Poloxamer-184 | Uniqema |
| Tego Carbomer 140 | Carbomer | Degussa |
| Texapon^{®} SB 3 | Aqua, Disodium Laureth Sulfosuccinate, (ca. 40% Aktivsubstanz), Citric Acid | Cognis |
| Tween^{®}-80 | Polysorbate-80 | |
| Uvinul MBC 95 | 4-METHYLBENZYLIDENE CAMPHOR | BASF |

## Patentansprüche

1. Kosmetische oder dermatologische Zusammensetzung zur topischen Behandlung der Haut, enthaltend in einem geeigneten kosmetischen oder dermatologischen Träger mindestens ein Aporphin-Alkaloid und mindestens einen weiteren Wirkstoff, ausgewählt aus
a) Purin und Purin-Derivaten,
b) natürlichen Betainverbindungen,
c) Harnstoff und alkyl- oder hydroxyalkylsubstituierten Harnstoffen,
d) Monomeren, Oligomeren und Polymeren von Aminosäuren, N-C₂-C₂₄-Acylaminosäuren und/oder den Estern und/oder den physiologisch verträglichen Salzen dieser Substanzen,
e) Polysacchariden,
f) hyperämisierenden Wirkstoffen, sowie
g) Mischungen der Substanzen a) - f).

2. Zusammensetzung gemäß Anspruch 1, **dadurch gekennzeichnet, dass** das Aporphin-Alkaloid ausgewählt ist aus Verbindungen der allgemeinen Struktur (APO-ALK-I), 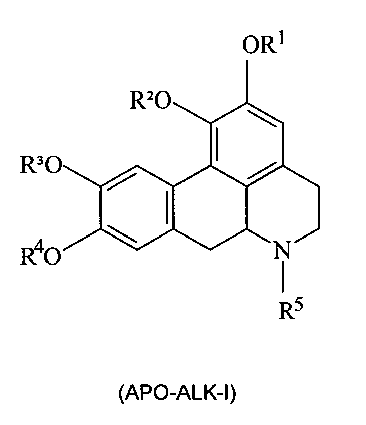 in der die Reste R¹, R², R³, R⁴ und R⁵, die gleich oder verschieden sein können, ausgewählt sind aus einem Wasserstoffatom, einem Halogenatom, einem C₁- bis C₄-Alkylrest, einem C₁-bis C₄-Monohydroxyalkylrest, einem C₂- bis C₄-Polyhydroxyalkylrest, einem (C₁- bis C₄)-Alkoxy-(C₁- bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁-bis C₄)-amino-alkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe, einer Aryl-C₁-C₆-alkylgruppe, einer Acylgruppe, einer Sulfonylgruppe oder einem Zucker, und den kosmetisch verträglichen Salzen von (APO-ALK-I) mit einer Säure, in Form aller 15 optischen Isomere, in isomerenreiner Form oder als Mischung optischer Isomere.

3. Zusammensetzung gemäß einem der vorstehenden Ansprüche, **dadurch gekennzeichnet, dass** das Aporphin-Alkaloid ausgewählt ist aus Verbindungen der allgemeinen Struktur (APO-ALK-I), in denen R¹ = R² = R³ = R⁴ = R⁵ = CH₃ ist.

4. Zusammensetzung gemäß einem der vorstehenden Ansprüche, **dadurch gekennzeichnet, dass** das Purin-Derivat ausgewählt ist aus Verbindungen der allgemeinen Struktur (PUR-I), 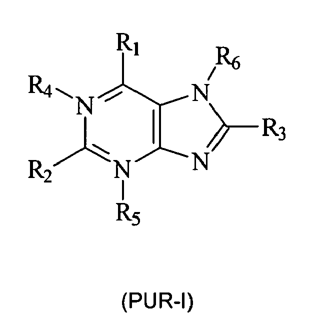 in der die Reste R¹, R² und R³ unabhängig voneinander ausgewählt sind aus -H, -OH, -NH₂, - SH und die Reste R⁴, R⁵ und R⁶ unabhängig voneinander ausgewählt sind aus -H, einem C₁-bis C₄-Alkylrest, einem C₁- bis C₄-Monohydroxyalkylrest, einem C₂- bis C₄-Polyhydroxyalkylrest, einem (C₁- bis C₄)-Alkoxy-(C₁- bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁-bis C₄)-aminoalkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe und einer Aryl-C₁-C₆-alkylgruppe.

5. Zusammensetzung gemäß einem der vorstehenden Ansprüche, **dadurch gekennzeichnet, dass** das Purin-Derivat ausgewählt ist aus Verbindungen der allgemeinen Struktur (PUR-II), 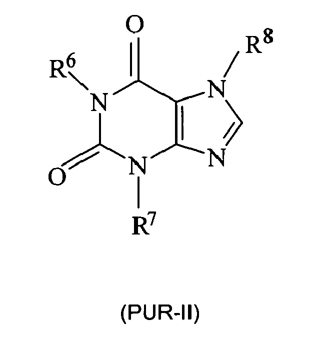 wobei in der Formel (PUR-II) die Reste R⁶, R⁷ und R⁸, die gleich oder verschieden sein können, ausgewählt sind aus einem Wasserstoffatom, einem C₁-C₄-Alkylrest, einem C₁-C₄-Mono-hydroxyalkylrest, einem C₂-C₄-Polyhydroxyalkylrest, einem (C₁-C₄)-Alkoxy-(C₁-C₄)-alkylrest, einem C₁-C₄-Aminoalkylrest, einem Hydroxy-(C₁-C₄)-alkylaminorest, einem C₁-C₄-Hydroxy-alkoxyrest, einem C₁-C₄-Hydroxyalkyl-(C₁-C₄)-aminoalkylrest oder einem (Di-C₁-C₄-Alkylamino)-(C₁-C₄)-alkylrest, einer gegebenenfalls substituierten Arylgruppe, insbesondere einer Phenylgruppe, einer gegebenenfalls substituierten Heteroarylgruppe und einer Aryl-C₁-C₆-alkylgruppe.

6. Zusammensetzung gemäß einem der vorstehenden Ansprüche, **dadurch gekennzeichnet, dass** das Purin-Derivat ausgewählt ist aus Verbindungen der allgemeinen Struktur (PUR-II), in denen R⁶ = R⁷= R⁸ = CH₃ (Coffein), oder R⁶ = H und R⁷ = R⁸ = CH₃ (Theobromin) oder R⁸ = H und R⁶ = R⁷ = CH₃ (Theophyllin) ist.

7. Zusammensetzung gemäß einem der vorstehenden Ansprüche, **dadurch gekennzeichnet, dass** die mindestens eine natürliche Betainverbindung ausgewählt ist aus Betain (Me₃N⁺-CH₂-COO⁻) und Carnitin (Me₃N⁺-CH₂-CHOH-CH₂-COO⁻), jeweils mit Me = Methyl.

8. Zusammensetzung gemäß einem der vorstehenden Ansprüche, **dadurch gekennzeichnet, dass** die alkyl- oder hydroxyalkylsubstituierte Harnstoffverbindung ausgewählt ist aus Verbindungen der allgemeinen Struktur (UREA-I), 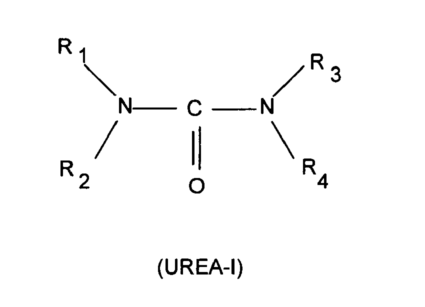 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-Cₛ-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ bis R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.

9. Zusammensetzung gemäß einem der vorstehenden Ansprüche, **dadurch gekennzeichnet, dass** die alkyl- oder hydroxyalkylsubstituierte Harnstoffverbindung Bis-N,N'-(2-Hydroxyethyl)harnstoff ist.

10. Zusammensetzung gemäß einem der vorstehenden Ansprüche, **dadurch gekennzeichnet, dass** das mindestens eine Monomer der Aminosäuren und/oder der N-C₂-C₂₄-Acylaminosäuren ausgewählt ist aus Alanin, Arginin, Asparagin, Asparaginsäure, Canavanin, Citrullin, Cystein, Cystin, Dipalmitoylhydroxyprolin, Desmosin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat.

11. Zusammensetzung gemäß einem der vorstehenden Ansprüche, **dadurch gekennzeichnet, dass** das mindestens eine Oligomer der Aminosäuren und/oder der N-C₂-C₂₄-Acylaminosäuren ausgewählt ist aus Peptiden mit 2 - 30, bevorzugt 2 - 15, Aminosäuren, bevorzugt ausgewählt aus Di-, Tri-, Tetra-, Penta-, Hexa- oder Pentadecapeptiden, die N-acyliert und/oder verestert sein können.

12. Zusammensetzung gemäß einem der vorstehenden Ansprüche, **dadurch gekennzeichnet, dass** das mindestens eine Polymer der Aminosäuren und/oder der N-C₂-C₂₄-Acylaminosäuren ausgewählt ist aus pflanzlichen und tierischen Proteinen und/oder Proteinhydrolysaten, die kationisiert sein können, sowie aus DNA-Reparaturenzymen.

13. Zusammensetzung gemäß einem der vorstehenden Ansprüche, **dadurch gekennzeichnet, dass** das mindestens eine Polysaccharid ausgewählt ist aus Polysacchariden, die mindestens einen Desoxyzucker-Baustein, insbesondere Fucose und/oder Rhamnose, enthalten, aus den Glycosaminoglycanen, besonders bevorzugt Hyaluronsäure, Dextran, Dextransulfat, Chondroitin-4-sulfat und Chondroitin-6-sulfat, weiterhin ausgewählt aus den Glucanen (Polyglucosanen), insbesondere Cellulose, Amylose, Glycogen, Lichenin, Laminarin aus Algen, Pachyman aus Baumpilzen und Hefeglucanen mit β-1,3-Bindung; Nigeran, und Pustulan (β-1,6-Glucan), Curdlan (β-1,3-D-Glucan), Pullulan (α-1,4-gebundenes und α-1,6-gebundenes D-Glucan) und Schizophyllan (β-1,3-Grundkette, β-1,6-Seitenkette), weiterhin ausgewählt aus beta-(1,3-1,4)-Glucanen, chemisch modifizierten Cellulose-Derivaten, vorzugsweise Hydroxyalkylcellulosen wie Hydroxyethylcellulose, Hydroxypropylcellulose, Hydroxypropylmethylcellulose, Methylcellulose, Hydroxymethylcellulose, Carboxymethylcellulose, weiterhin insbesondere aus Stärken sowie Stärkeabbauprodukten wie Amylose und Amylopektin, chemisch und/oder thermisch modifizierte Stärken, z. B. Hydroxypropylstärkephosphat oder Dihydroxypropyldistärkephosphat, weiterhin Chitosan und dessen Salzen und anderen Derivaten, weiterhin aus Polysacchariden, die Gums oder Gummen bilden, wie Guar-Gum, Xanthan-Gum, Alginate, insbesondere Natriumalginat, Gummi arabicum, Karaya-Gummi, Carrageenane, Johannisbrotkernmehl, Leinsamen-Gums, Schellack und Agar-Agar.

14. Zusammensetzung gemäß einem der vorstehenden Ansprüche, **dadurch gekennzeichnet, dass** der mindestens eine hyperämisierende Wirkstoff ausgewählt ist aus
- Nicotinsäureestern, insbesondere Nicotinsäurebenzylester (Benzylnicotinat), Nicotinsäurebutoxyethylester (Nicoboxil), Nicotinsäuremethylester, Nicotinsäureethylester, Nicotinsäurehexylester, Nicotinsäureisopropylester, Nicotinsäuremyristylester, Tetrahydrofurfuryl-Nicotinat (Thurfyl-Nicotinat) und Tetrahydrofurfuryl-Nicotinat-Hydrochlorid,
- Pyridin-3-carbaldehyd (β-Pyridincarbaldehyd, Nicotinaldehyd),
- Salicylsäureestern, insbesondere Phenylsalicylat,
- Vanillylethern, insbesondere Vanillylbutylether,
- Carbonsäurevanillylamiden, insbesondere Nonivamid,
- Scharfstoffen, insbesondere Capsaicin, Cantharidin, Piperin, Gingerol, Zingeron, Myristicin, Safrol, Allicin, Sedamin, Sacculatal, Chalciporon, Isochalciporon, Velleral, Vellerol, und Isothiocyanaten (Senfölen), insbesondere Benzylsenföl,
- Extrakten aus Scharfstoff-Pflanzen, insbesondere aus Capsicum, insbesondere aus Capsicum annuum und Capsicum frutescens, aber auch aus Capsicum chinense, Capsicum baccatum und Capsicum pubescens, weiterhin aus Mauerpfeffer und aus der Zaunrübe,
- Terpentinöl,
- sowie Mischungen hiervon.

15. Zusammensetzung gemäß einem der vorstehenden Ansprüche, **dadurch gekennzeichnet, dass** mindestens ein die Collagensynthese stimulierender Wirkstoff enthalten ist, ausgewählt aus
- Retinoiden, insbesondere Retinol und C₂-C₂₂-Fettsäureestern des Retinols,
- Kombucha,
- Olivenblattextrakten (Olea Europaea (Olive) Leaf Extract),
- Oleanolsäure,
- Oleanol,
- Grüntee-Extrakten (Camellia Sinensis),
- Kreatin,
- sowie Mischungen der vorgenannten Substanzen.

16. Zusammensetzung gemäß einem der vorstehenden Ansprüche, **dadurch gekennzeichnet, dass** mindestens ein die Interaktion zwischen der extrazellulären Matrix und den Fibroblasten erhöhender und/oder verbessernder Wirkstoff enthalten ist, ausgewählt aus
- Apfelkernextrakten (Pyrus Malus (Apple) Fruit Extract),
- Lotuskeim-Extrakten (Nelumbo Nucifera Germ Extract),
- Extrakten aus Maiskörnern (Zea Mays (Corn) Kernel Extract),
- Rotweinextrakten,
- Traubenkernextrakten (Vitis Vinifera (Grape) Seed Extract), die bevorzugt aus der Chardonnay-Traube stammen,
- Extrakten aus Schwarzen Holunderblüten (Sambucus Nigra Flower Extract),
- Mischungen aus mindestens einem Extrakt aus Kakaobohnen (Theobroma cacao) und mindestens einem Extrakt aus den Blättern der Pfefferminze (Mentha piperita),
- Hydroxystilbenen und deren Estern, insbesondere Resveratrol und/oder Resveratrolmono-, -di- und -triphosphorsäureestern und deren Salzen, weiterhin aus Piceatannol, Piceatannolethern und Pinosylvin sowie Pinosylvinethern
- Isoflavonoiden und Isoflavonoid-reichen Pflanzenextrakten,
- Dihydroquercetin (= Taxifolin),
- sowie Mischungen hiervon.

17. Zusammensetzung gemäß einem der vorstehenden Ansprüche, **dadurch gekennzeichnet, dass** mindestens ein weiterer Wirkstoff enthalten ist, ausgewählt aus
- DNA- oder RNA-Oligonucleotiden,
- Vitaminen, Provitaminen und Vitaminvorstufen der Gruppen B, E, H und K und den Estern der vorgenannten Substanzen,
- α-Hydroxycarbonsäuren, α-Ketocarbonsäuren, β-Hydroxycarbonsäuren und deren Ester-, Lacton- und/oder Salzform,
- Flavonoiden und Flavonoid-reichen Pflanzenextrakten,
- Ubichinon und Ubichinol sowie deren Derivaten,
- Silymarin,
- Ectoin,
- anorganischen und organischen UV-Filtersubstanzen,
- selbstbräunenden,
- hautberuhigenden und
- sebumregulierenden Wirkstoffen sowie
- Mischungen dieser Wirkstoffe.

18. Zusammensetzung gemäß einem der vorstehenden Ansprüche, **dadurch gekennzeichnet, dass** mindestens ein nichtionischer Emulgator mit einem HLB-Wert von 8 und darunter der allgemeinen Formel R¹ - O - R², in der R¹ eine primäre lineare Alkyl-, Alkenyl- oder Acylgruppe mit 20 - 30 Kohlenstoffatomen, bevorzugt einen Behenyl-Rest, und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen, bevorzugt Wasserstoff, darstellt, bevorzugt Behenylalkohol, enthalten ist.

19. Zusammensetzung gemäß einem der vorstehenden Ansprüche, **dadurch gekennzeichnet, dass** sie in Form einer lamellare Strukturen aufweisenden Öl-in-Wasser-Emulsion vorliegt.

20. Zusammensetzung gemäß einem der vorstehenden Ansprüche, **dadurch gekennzeichnet, dass** sie in Form einer Wasser-in-Öl-Emulsion vorliegt.

21. Zusammensetzung gemäß einem der vorstehenden Ansprüche, **dadurch gekennzeichnet, dass** sie in einem Sprühspender oder Pumpspender verpackt ist.

22. Nicht-therapeutische, kosmetische Verwendung einer kosmetischen oder dermatologischen Zusammensetzung gemäß einem der Ansprüche 1 - 21zur Anti-Cellulite-Behandlung der Haut und/oder zur Steigerung der Hautfestigkeit und/oder zur Verbesserung der mechanischen Stabilität der Haut und/oder zur Steigerung der Hautelastizität und/oder der Hautstraffheit und/oder zur Glättung der Haut und/oder zur Verbesserung des optischen Erscheinungsbildes der Haut und/oder zur Anregung der Mikrozirkulation in der Haut und/oder zur Unterstützung der Lipolyse in der Subcutis und/oder zur Inhibierung der Lipogenese in der Subcutis.

23. Verwendung einer Wirkstoffkombination aus mindestens einem Aporphin-Alkaloid und mindestens einem weiteren Wirkstoff, ausgewählt aus
a) Purin-Alkaloiden,
b) Monomeren, Oligomeren und Polymeren von Aminosäuren, N-C₂-C₂₄-Acylaminosäuren und/oder den Estern und/oder den physiologisch verträglichen Salzen dieser Substanzen, sowie Mischungen dieser Wirkstoffe,
c) natürlichen Betainverbindungen,
d) Harnstoff und N-Alkyl-substituierten Harnstoffderivaten,
e) Polysacchariden,
f) Hyperämisierenden Wirkstoffen, sowie
g) Mischungen der Wirkstoffe a) - f),
zur Herstellung einer kosmetischen oder dermatologischen Zusammensetzung
- zur Behandlung der Cellulite und/oder
- zur Steigerung der Hautfestigkeit und/oder
- zur Verbesserung der mechanischen Stabilität der Haut und/oder
- zur Steigerung der Hautelastizität und/oder
- zur Straffung der Haut und/oder
- zur Glättung der Haut und/oder
- zur Verbesserung des optischen Erscheinungsbildes der Haut
- zur Anregung der Mikrozirkulation in der Haut und/oder
- zur Unterstützung der Lipolyse in der Subcutis und/oder
- zur Inhibierung der Lipogenese in der Subcutis.
